# EUROPEAN PATENT SPECIFICATION

(11) **EP 3 481 406 B1**
(45) Date of publication and mention of the grant of the patent: **05.06.2024**
(21) Application number: 17740070.2
(22) Date of filing: 06.07.2017
(51) Int. Cl.: A61K 35/747, A61K 31/702, A61K 31/366, A61K 31/51, A61K 31/575, A61K 31/716, A61K 31/732, A61K 33/00, A61K 33/24, A61P 9/12, A61P 9/00, A61P 3/00, A61P 3/06, A61K 31/202, A61K 31/40, A61K 31/455, A61K 31/722, A61K 31/733, A61K 36/18, A61P 43/00

(54) **COMPOSITION**
ZUSAMMENSETZUNG
COMPOSITION

(30) Priority: 06.07.2016 GB 201611782
(43) Date of publication of application: 15.05.2019
(73) Proprietor: Probiotix Health Limited, Pontefract WF8 4HH (GB)
(72) Inventor: O'HARA, Stephen Patrick, Heslington York YO10 5DG (GB); KOLIDA, Sofia, Heslington York YO10 5DG (GB); GOSALBEZ, Luis, Heslington York YO10 5DG (GB)
(74) Representative: Appleyard Lees IP LLP
(86) International application number: PCT/GB2017/051993
(87) International publication number: WO 2018/007820

(56) References cited:
- WO-A1-2015/067947
- WO-A1-2015/149818
- WO-A1-2017/077285
- US-A1- 2005 239 871
- ULRIKA AXLING ET AL: "Green tea powder and Lactobacillus plantarum affect gut microbiota, lipid metabolism and inflammation in high-fat fed C57BL/6J mice", NUTRITION & METABOLISM, BIOMED CENTRAL. LONDON, GB, vol. 9, no. 1, 26 November 2012 (2012-11-26), page 105, XP021137109, ISSN: 1743-7075, DOI: 10.1186/1743-7075-9-105
- SCHAAFSMA G ET AL: "Effects of a milk product, fermented by Lactobacillus acidophilus and with fructo-oligosaccharides added, on blood lipids in male volunteers.", EUROPEAN JOURNAL OF CLINICAL NUTRITION JUN 1998, vol. 52, no. 6, June 1998 (1998-06), pages 436-440, XP009500285, ISSN: 0954-3007
- LIONG M T ET AL: "Optimization of cholesterol removal, growth and fermentation patterns of Lactobacillus acidophilus ATCC 4962 in the presence of mannitol, fructo-oligosaccharide and inulin: a response surface methodology approach", JOURNAL OF APPLIED MICROBIOLOGY, WILEY-BLACKWELL PUBLISHING LTD, GB, vol. 98, no. 5, 30 April 2005 (2005-04-30) , pages 1115-1126, XP009500289, ISSN: 1364-5072, DOI: 10.1111/J.1365-2672.2005.02544.X [retrieved on 2005-02-28]
- DATABASE WPI Week 201437 Thomson Scientific, London, GB; AN 2014-K25349 XP002773982, & CN 103 689 595 A (UNIV TIANJIN SCI & TECHNOLOGY) 2 April 2014 (2014-04-02)

## Description

### Technical Field

The disclosure relates to compositions comprising *Lactobacillus plantarum* in combination with one or more active ingredients for use in the treatment, prevention or control of blood lipid levels (e.g. cholesterol, triglycerides) and/or management of hypertension.

### Background

Cardiovascular disease (CVD) is a leading cause of death globally. The World Health Organisation (WHO) predicts that by the year 2020, up to 40% of all human deaths will be related to CVD. Elevated blood lipid levels, in particular raised levels of low density lipoprotein cholesterol (LDL-C), are known risk factors for CVD and coronary artery disease (CAD). There is increasing evidence that high triglyceride levels may contribute to hardening of the arteries or thickening of the artery walls (atherosclerosis) and represent an important biomarker of cardiovascular risk.

Therapies for the reduction and control of blood lipid levels and specifically LDL-C and triglyceride levels are being researched intensively.

Hypertension, otherwise known as high blood pressure, is a major health problem and is a risk factor for CVD. The World Health Organisation (WHO) predicts that by the year 2020, up to 40% of all human deaths will be related to CVD. High cholesterol is often linked to hypertension due to a build-up of cholesterol plaque in blood vessels.

The majority of cholesterol-lowering therapies currently used are statins. However, statins have a range of intolerance and safety concerns which affect compliance and they are expensive. Plant sterols and stanols have been explored as possible alternatives to statins. However large amounts of these substances, 3-4 tea spoons, need to be taken to achieve an average reduction in LDL-C of between 7 and 10.5 %. This is an issue as plant sterols and stanols are expensive.

There has been increasing interest in non-drug therapies such as probiotics to improve blood cholesterol profiles. A number of studies have identified the role of bile acids as signalling molecules in regulating lipid, glucose, and energy metabolism. Bile acids transport dietary fat and cholesterol into the circulation. The use of microbial strains in the reduction of cholesterol levels, and therefore potentially hypertension, by regulating bile acid regulators is known. Bile Salt Hydrolase (BSH) active probiotics have been shown to increase intraluminal bile acid deconjugation, resulting in increased levels of circulating deconjugated bile salts in humans and animal studies. As bile acids are deconjugated in the intestines, dietary and biliary cholesterol absorption is reduced and the recirculation of bile is altered, resulting in better control of (LDL-C) levels in blood. A number of studies have tested and shown the lipid lowering effects of a probiotic in commercial yoghurts.

Studies have suggested a role of probiotics in reducing blood pressure. A meta-analysis of nine trials *(*Khalesi et. al., (2014), Hypertension, 64, (4), 897-903) showed probiotic consumption changed systolic BP by -3.56 mm Hg (95% confidence interval, -6.46 to -0.66) and diastolic BP by -2.38 mm Hg (95% confidence interval, -2.38 to -0.93) compared with control groups.

In WO2015/067947, *Lactobacillus plantarum* strains have been suggested as BSH active probiotics with high upper gastrointestinal survival characteristics.

The Derwent World Patents Index abstract of CN 103689595 discloses a lactic acid bacteria soft capsule and states that it improves intestinal channel function, immunity and "reduces cholesterol and blood pressure".

It is an object of the present disclosure to provide an improved or alternative treatment for the management of hypertension and in doing so reduce cardiovascular risk.

It is a further object of the present disclosure to provide a probiotic composition which can be employed to help maintain the correct blood pressure in an individual. It would also be beneficial if the probiotic composition could be used alongside existing functionally or pharmaceutically active ingredients, and act synergistically or have different but complementary modes of action. It would be desirable if the probiotic composition could be used to enable lower doses of the existing cholesterol and/or triglyceride reduction ingredients to be utilised or to enhance overall health benefits.

### Summary

The present invention is defined by the appended claims.

The components of the composition advantageously work synergistically.

The one or more active ingredients may further comprise one or more lipid modifying active ingredients selected from the following: statins; stanols and sterols; monacolin K; fatty acids; niacin, potassium, soluble fibres; and/or prebiotics.

The statin may be selected from one or more of the following: fluvastatin; pravastatin; simvastatin; atorvastatin; and/or rosuvastatin. It is envisaged that the present disclosure would also be compatible with future statins which may be developed.

The fatty acid may be selected from one or more of the following: alpha-linolenic acid (ALA); omega-3 polyunsaturated fatty acids (PUFAs); and/or omega-6 polyunsaturated fatty acids.

The soluble fibre may be selected from one or more of the following: β-glucans; pectin; and/or chitosan.

The prebiotic may be selected from one or more of the following: inulin; fructooligosaccharides (FOS); chitooligosaccharides (COS); xylooligosaccharides (XOS); gentiooligosaccharides; cellobiose and/or soyabean oligosaccharides.

The composition may be for use in the management, treatment or prevention of hypertension in an individual. The composition may be for use in the manufacture of a medicament for the treatment or prevention of hypertension. Preferably, the composition acts on more than one blood lipid parameter and to treat or prevent hypertension. Such compositions may comprise monacolin K for and *Lactobacillus plantarum* ECGC 13110402 for treating hypertension.

Compositions may comprise monacolin K and *Lactobacillus plantarum* ECGC 13110402. The components of compositions of the present disclosure have a combined effect of reducing overall cardiovascular risk more so than if a single component were administered alone.

Also disclosed is a composition comprising Lactobacillus plantarum ECGC 13110402, GOS, produced by the Lactobacillus plantarum strain and one or more cholesterol modifying active ingredients for use in the treatment or prevention of hypertension.

Also disclosed is a composition comprising Lactobacillus plantarum ECGC 13110402, GOS, produced by the Lactobacillus plantarum strain and one or more cholesterol modifying active ingredients, for use in the manufacture of a food supplement or food ingredient for management, treatment or prevention of hypertension.

Preferably, *Lactobacillus plantarum* ECGC 13110402 will be administered to an individual in an amount in the range of 10⁵ cfu to 10¹² cfu per dose. More preferably, *Lactobacillus plantarum* ECGC 13110402 may be in an amount in the range of 10⁸ cfu to 10¹⁰ cfu per dose. Although it will be appreciated that different dosages may be administered depending upon the individuals' condition. Even more preferably, the *Lactobacillus plantarum* is in an amount in the range of about 50mg to about 150 mg of the active strain providing about 1.8 × 10⁹ cfu per dose. Most preferably, the *Lactobacillus plantarum* is in an amount of about 120mg of the active strain providing about 1.8 × 10⁹ cfu per dose. It is envisaged that the dose will be administered either once or twice daily.

The composition may provide *Lactobacillus plantarum* ECGC 13110402 in a dose in the range of 2 × 10⁵ to 2 × 10¹² cells. More preferably, the *Lactobacillus plantarum* is present in a dose in the range of 2 × 10⁸ to 2 × 10¹⁰ cells. Even more preferably, the *Lactobacillus plantarum* is present in a dose in the range of about 100 to about 300 mg of the active strain providing about 2.6 × 10⁹ cells. Most preferably, the *Lactobacillus plantarum* is present in a dose in the range of about 120 mg of the active strain providing about 1.8 × 10⁹ cells per dose. The dose may be administered either once or twice daily.

The composition may provide *Lactobacillus plantarum* ECGC 13110402 in a daily dose in the range of 2 × 10⁵ to 2 × 10¹² cells. More preferably, the *Lactobacillus plantarum* is present in a daily dose in the range of 2 × 10⁸ to 2 × 10¹⁰ cells. Even more preferably, the *Lactobacillus plantarum* is present in a daily dose in the range of about 100 to about 300 mg of the active strain providing about 2.6 × 10⁹ cells. Most preferably, the *Lactobacillus plantarum* is present in a daily dose in the range of about 120 mg of the active strain providing about 1.8 × 10⁹ cells.

The one or more active ingredients may comprise fluvastatin in a daily dose in the range of about 20 mg to about 80 mg; pravastin in the range of about 10 mg to about 40 mg; simvastin in a daily dose in the range of about 10 mg to about 80 mg; rosuvastin in a daily dose in the range of about 5 mg to about 40 mg; and/or atorvastin in a daily dose in the range of about 10 mg to about 80 mg.

If a low intensity action is desired, then the composition may comprise fluvastatin in a daily dose in the range of about 20 mg to about 40 mg; pravastatin in a daily dose in the range of about 10 mg to about 40 mg; or simvastatin in a daily dose of about 10 mg.

If a medium intensity action is desired, then the composition may comprise atorvastatin in a daily dose of about 10 mg; fluvastatin in a daily dose of about 80 mg; rosuvastatin in a daily dose of about 5 mg; or simvastatin in a daily dose in the range of about 20 to about 40 mg.

If a high intensity action is desired, then the composition may comprise atorvastatin in a daily dose in the range of about 20 to about 80 mg; rosuvastatin in a daily dose in the range of about 10 to about 40 mg; or simvastatin in a daily dose of about 80 mg.

One of the advantages of the present disclosure is that the provision of *Lactobacillus plantarum* ECGC 13110402 enables lower doses of cholesterol modifying active ingredients, such as statins, to be used. Therefore, the one or more active ingredients may comprise fluvastatin in a daily dose of up to about 20 mg; pravastin in a daily dose of up to about 10 mg; simvastin in a daily dose of up to about 10 mg; rosuvastin in a daily dose of up to about 5 mg; and/or atorvastin in a daily dose of up to about 10 mg. More preferably, the statins are used in a lower dose and therefore the one or more active ingredients may comprise fluvastatin in a daily dose of up to about 18 mg; pravastin in a daily dose of up to about 8 mg; simvastin in a daily dose of up to about 8 mg; rosuvastin in a daily dose of up to about 3 mg; and/or atorvastin in a daily dose of up to about 8 mg.

The one or more active ingredients may comprise plant stanols and sterols in a daily dose in the range of about 0.7 g to about 3 g. More preferably, the one or more active ingredients may comprise plant stanols and sterols in a daily dose in the range of about 0.7 g to about 2.5 g. Even more preferably, the stanols and sterols are used in a lower dose and therefore the one or more active ingredients may comprise plant stanols and sterols in a daily dose of up to about 0.7 g, more preferably up to about 0.6 g, and even more preferably up to about 0.5 g.

The one or more active ingredients comprise monacolin K in a daily dose in the range of about 2.5 mg to about 15 mg or to about 10 mg. More preferably, monacolin K is used in a lower dose and therefore the one or more active ingredients may comprise monacolin K in a daily dose of up to about 2.5 mg, more preferably up to about 2.3 mg, and even more preferably up to about 2 mg.

The one or more active ingredients may comprise fatty acids comprising ALA in a daily dose in the range of about 0.1 g to about 3.5 g. Preferably, the one or more active ingredients are used at doses in the lower range and therefore may comprise a daily dose of ALA in the range of about 0.1 g to about 2 g. Most preferably, the one or more active ingredients comprise fatty acids comprising a daily dose of ALA in the range of about 0.1 g to about 1 g. Alternatively, the one or more active ingredients may comprise fatty acids comprising ALA in a daily dose of up to about 3.5 g. More preferably, the one or more active ingredients may comprise fatty acids comprising ALA in a daily dose of up to about 0.1 g. Most preferably, the one or more active ingredients may comprise fatty acids comprising ALA in a daily dose of up to about 0.05 g.

The one or more active ingredients may comprise fatty acids comprising Omega-3 PUFAs in a daily dose in the range of about 0.5 g to about 4 g. Preferably, the one or more active ingredients are used at doses in the lower range and therefore may comprise a daily dose of Omega-3 PUFAs in the range of about 1 g to about 4 g. Most preferably, the one or more active ingredients comprise fatty acids comprising a daily dose of Omega-3 PUFAs in the range of about 1 g to about 2 g. Alternatively, the one or more active ingredients may comprise fatty acids comprising Omega-3 PUFAs in a daily dose of up to about 4 g. More preferably, the one or more active ingredients may comprise fatty acids comprising Omega-3 PUFAs in a daily dose of up to about 3 g. Even more preferably, the one or more active ingredients may comprise fatty acids comprising Omega-3 PUFAs in a daily dose of up to about 1 g. More preferably, the one or more active ingredients may comprise fatty acids comprising Omega-3 PUFAs in a daily dose of up to about 0.5 g or about 0.25 g.

The one or more active ingredients may comprise fatty acids comprising Omega-6 PUFAs in a daily dose in the range of about 1 g to about 60 g. Preferably, the one or more active ingredients are used at doses in the lower range and therefore may comprise a daily dose of Omega-6 PUFAs in the range of about 1 g to about 30 g. Most preferably, the one or more active ingredients comprise fatty acids comprising a daily dose of Omega-6 PUFAs in the range of about 1g to about 10 g. Alternatively, the one or more active ingredients may comprise fatty acids comprising Omega-6 PUFAs in a daily dose of up to about 60 g. More preferably, the one or more active ingredients may comprise fatty acids comprising Omega-6 PUFAs in a daily dose of up to about 10 g. Even more preferably, the one or more active ingredients may comprise fatty acids comprising Omega-6 PUFAs in a daily dose of up to about 1 g. Most preferably, the one or more active ingredients may comprise fatty acids comprising Omega-6 PUFAs in a daily dose of up to about 0.5 g.

The one or more active ingredients may comprise a daily dose of β-glucans in the range of about 2 g to about 10 g. Preferably, the one or more active ingredients comprises a daily dose of β-glucans in the range of about 2 g to about 6 g. More preferably, the one or more active ingredients comprises a daily dose of β-glucans in the range of about 2 g to about 4 g. Even more preferably, the one or more active ingredients comprises a daily dose of β-glucans up to about 3 g. Alternatively, the one or more active ingredients may comprise a daily dose of β-glucans of up to about 10 g. Preferably, the one or more active ingredients may comprise a daily dose of β-glucans of up to about 8 g. More preferably, the one or more active ingredients may comprise a daily dose of β-glucans of up to about 7 g.

The one or more active ingredients may comprise a daily dose of pectin in the range of about 1 g to about 10 g. Preferably, the one or more active ingredients comprises a daily dose of pectin in the range of about 1 g to about 6 g. More preferably, the one or more active ingredients comprises a daily dose of pectin in the range of about 1 g to about 4 g. Most preferably, the one or more active ingredients comprises a daily dose of pectin in the range of about 1 g to about 3 g. Alternatively, the one or more active ingredients may comprise a daily dose of pectin of up to about 10 g. Preferably, the one or more active ingredients comprises a daily dose of pectin of up to about 9 g. More preferably, the one or more active ingredients comprises a daily dose of pectin of up to about 2 g. Most preferably, the one or more active ingredients comprises a daily dose of pectin of up to about 1.75 g.

The one or more active ingredients may comprise a daily dose of pysllium in the range of about 5 g to about 15 g. Preferably, the one or more active ingredients comprises a daily dose of pysllium in the range of about 5 g to about 10 g. More preferably, the one or more active ingredients comprises a daily dose of pysllium in the range of about 5 g to about 8 g. Alternatively, the one or more active ingredients may comprise a daily dose of pysllium of up to about 15 g. Preferably, the one or more active ingredients comprises a daily dose of pysllium of up to about 10 g. More preferably, the one or more active ingredients comprises a daily dose of pysllium of up to about 7 g.

The one or more active ingredients may comprise a daily dose of chitosan in the range of about 1 g to about 5 g. Preferably, the one or more active ingredients comprises a daily dose of chitosan in the range of about 1 g to about 3 g. More preferably the one or more active ingredients comprises a daily dose of chitosan in the range of about 2 g to about 3 g. Alternatively, the one or more active ingredients may comprise a daily dose of chitosan of up to about 5 g. Preferably, the one or more active ingredients comprises a daily dose of chitosan of up to about 4 g. More preferably, the one or more active ingredients comprises a daily dose of chitosan of up to about 3 g.

The one or more active ingredients may comprise a daily dose of inulin in the range of about 5 g to about 20 g. Preferably the one or more active ingredients comprises a daily dose of inulin in the range of about 5 g to about 20 g. More preferably, the one or more active ingredients comprises a daily dose of inulin in the range of about 10 g to about 20 g. Alternatively, the one or more active ingredients may comprise a daily dose of inulin of up to about 20 g. Preferably, the one or more active ingredients comprises a daily dose of inulin of up to about 15 g. More preferably, the one or more active ingredients comprises a daily dose of inulin of up to about 10 g. Even more preferably, the one or more active ingredients comprises a daily dose of inulin of up to about 5 g.

The one or more active ingredients may comprise a daily dose of fructooligosaccharides (FOS) in the range of about 5 g to about 15 g. Preferably, the one or more active ingredients comprises a daily dose of FOS in the range of about 5 g to about 12 g. More preferably, the one or more active ingredients comprises a daily dose of FOS in the range of about 5 g to about 10 g. Most preferably, the one or more active ingredients comprises a daily dose of FOS in the range of about 5 g to about 8 g. Alternatively, the one or more active ingredients may comprise a daily dose of FOS of up to about 15 g. Preferably, the one or more active ingredients comprises a daily dose of FOS of up to about 12 g. More preferably, the one or more active ingredients comprises a daily dose of FOS of up to about 10 g. Most preferably, the one or more active ingredients comprises a daily dose of FOS of up to about 8 g. Most preferably, the one or more active ingredients comprises a daily dose of FOS of up to about 5 g.

The composition may comprise a daily dose of galactooligosaccharides (GOS) in the range of about 10 g to about 20 g. Preferably, the one or more active ingredients comprises a daily dose of GOS in the range of about 10 g to about 17 g. More preferably, the one or more active ingredients comprises a daily dose of GOS in the range of about 10 g to about 15 g. Most preferably, the one or more active ingredients comprises a daily dose of GOS in the range of about 5 g to about 10 g. Alternatively, the one or more active ingredients may comprise a daily dose of GOS of up to about 20 g. Preferably, the one or more active ingredients comprises a daily dose of GOS of up to about 17 g. More preferably, the one or more active ingredients comprises a daily dose of GOS of up to about 15 g. Most preferably, the one or more active ingredients comprises a daily dose of GOS of up to about 12 g. Most preferably, the one or more active ingredients comprises a daily dose of GOS of up to about 10 g.

The composition may comprise further excipients necessary for the manufacture of a dosage form and its breakdown following ingestion. The composition may further comprise disintegrants, binders, lubricants and glidants.

The composition may further comprise one or more disintegrants selected from: polyvinylpyrollidone, sodium starch glycolate and carboxymethylcellulose.

The composition may further comprise one or more binders selected from; starches, saccharides, cellulose, sugar alcohols, gelatin, polyvinylpyrollidone and polyethylene glycol. Preferably the composition further comprises corn starch.

The composition may further comprise one or more glidants selected from talc, magnesium carbonate, fumed silica and silicon dioxide. Preferably the composition further comprises silicon dioxide.

The composition may further comprise one or more lubricants selected from stearic acid, vegetable stearin and magnesium stearate. Preferably the composition further comprises magnesium stearate.

Administration frequency would also be dependent upon an individuals' condition but preferably the composition would be administered twice daily.

The composition may be administered at any time of day. However, preferably the composition is adminstered before meals.

It will be apparent to the skilled addressee that the composition may be in any easily administered form, for example in the form of a powder, tablet, or capsule. Alternatively, the composition may be in the form of a food stuff or food additive. The composition may be in the form of a drinkable liquid, a spread and/or powder which can be mixed with a solid or liquid food stuff. The composition could be used as a dietary supplement - for example to be blended with foods/drinks or consumed alongside foods/drinks.

The composition may further comprise an excipient or carrier compound to modify the release profile of one or more of the components through the intestinal environment. Release should occur at the most appropriate time for reducing cholesterol absorption. Typically, the culture must survive relatively intact until it reaches the intestinal enterocytes of the small intestine.

The composition may be encapsulated. Many encapsulation techniques will be apparent to the skilled addressee and the one employed will be tailored to the required stability of *Lactobacillus plantarum* ECGC 13110402 and/or the active ingreident during digestive transit.

The *Lactobacillus plantarum* ECGC 13110402 component may be concentrated and/or freeze dried. Advantageously *Lactobacillus plantarum* ECGC 13110402 has demonstrated excellent freeze drying survival in pilot scale manufacturing trials.

The composition may further comprise one or more active ingredients selected from: vitamins, minerals, phytochemicals, antioxidants, and combinations thereof.

Vitamins may include fat soluble vitamins such as vitamin A, vitamin D, vitamin E, and vitamin and combinations thereof. Vitamins can include water soluble vitamins such as vitamin C (ascorbic acid), the B vitamins (thiamine or B 1, riboflavoin or B25 niacin or B3, pyridoxine or B6, folic acid or B9, cyanocobalimin or B12, pantothenic acid, biotin), and combinations thereof.

Minerals may include, but are not limited to, sodium, magnesium, chromium, iodine, iron, manganese, calcium, copper, fluoride, potassium, phosphorous, molybdenum, selenium, zinc, and combinations thereof.

Antioxidants may include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, fiavonoids, and combinations thereof.

Phytochemicals may include but are not limited to cartotenoids, chlorophyll, chlorophyllin, fiber, flavanoids, anthocyamns, cyaniding, delphinidin, malvidin, pelargonidin, peonidin, petunidin, flavanols, catechin, epicatechin, epigallocatechin, epigailocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof.

The composition may comprise a prebiotic specifically tailored to *Lactobacillus plantarum* ECGC 13110402. The prebiotic may selectively accentuate the growth and survivability of *Lactobacillus plantarum* ECGC 13110402.

The composition may further comprise one or more fillers. The composition may further comprise one or more fillers selected from the following: maltodextrin, sucrose or fillers with cholesterol reducing ability. Preferably the composition further comprises beta glucans which can reduce cholesterol thus cooperatively enhancing the cholesterol reducing/controlling functions of the other excipients in the composition.

The composition may be administered with one or more statins, sterols and/or stanols. Advantageously co-administration with known cholesterol lowering therapeutics can provide enhanced cholesterol reduction and/or control. Plant sterols ahve been shown to increase levels of serum plant sterols which have been found part of atherosclerotic plaques and in the retina of long-term plant sterol and stanol users. BSH-active probiotic bacteria have been shown to reduce circulating cholesterol and plant sterols. A combination of plant sterols and BSH-active probiotics can therefore reduce/control cholesterol levels and reduce plant sterol serum levels advantageously improving the safety profile of sterol products. Mechanistically BSH-active bacteria should work in a complementary fashion with statins to amplify LDL receptor activity and the clearance of serum cholesterol, as they increase bile salt deconjugation and reduce sterol absorption. Therefore co-administration of BSH-active probiotics and statins can potentially result in a greater reduction in serum LDL-C enabling a reduction in statin dosage thus reducing costs and side effects and improving patient compliance.

Preferably the composition is stored at 4°C or below. Bacterial growth is stabilised in this temperature range thus ensuring the stability of the composition.

The composition may further comprise a prebiotic growth medium which is specific to the growth of the *Lactobacillus plantarum* strain. The prebiotic growth medium will preferably be capable of being producing by the *Lactobacillus plantarum* strain by reverse enzyme reaction. The enzyme may comprise a saccharolytic or glycosidase enzymes. These saccharolytic or glycosidase enzymes may be derived from bacteria or fungi.

The prebiotic growth medium may comprise oligosaccharides such as galacto-oligosacharides, (GOS), gluco-oligosacharides, or fructo- oligosaccharides (FOS) in varying concentrations. It is preferred that the oligosaccharide form is substantially the same as the form produced by β-galactosidases, α-galactosidases, α- and β-glucosidases, α-mannosidases and β-xylosidases reverse reactions of the strain.

The prebiotic growth medium may be present in an amount which provides optimal growth and survival of the strain within the gut without impacting on safety, tolerance, and shelf life.

It will be apparent to the skilled addressee that the composition may be manufactured in a number of different product formats and forms, such as in the form of a capsule, tablet, powder or a liquid. The composition herein above described may comprise the *L. Plantarum* in the range of about 2 billion to about 6 billion cells and one or more following: monacolin K in the range of about 2.5 mg to about 15 mg, vitamin B1 in the range of about 0.5 mg to 1.5 mg, vitamin B3 in the range of about 10 mg to 20 mg, plant stanols in the range of about 200 mg to about 600 mg, omega-3 fatty acids EPA and DHA in the range of about 200 mg to 300 mg, potassium in the range of about 100 mg to about 200 mg, and/or chromium in the range of about 10 µg to 50 µg.

More preferably, the composition comprises the *L. Plantarum* in the range of about 2.5 billion to about 5 billion cells and one or more following: monacolin K in the range of about 5 mg to about 10 mg, vitamin B1 at about 1 mg, vitamin B3 at about 16 mg, plant stanols at about 400 mg, omega-3 fatty acids EPA and DHA at about 250 mg, potassium at about 150 mg, and/or chromium at about 20 µg.

The *Lactobacillus plantarum* may be administered shortly before, during or after morning and evening meals. Preferably, the *Lactobacillus plantarum* is administered shortly before breakfast and the evening meal.

The *Lactobacillus plantarum* may be administered as a medicine or as a dietary supplement.

The *Lactobacillus plantarum* may be in a freeze dried form.

The *Lactobacillus plantarum* may be administered in combination with a prebiotic growth medium which is specific to the growth of the *Lactobacillus plantarum* strain. The prebiotic growth medium will preferably be capable of being producing by the *Lactobacillus plantarum* strain by reverse enzyme reaction. The prebiotic growth medium may comprise oligosaccharides, which will preferably comprise galacto-oligosaccharide (GOS). Preferably, the *Lactobacillus plantarum* is stored at 4°C or below before administration.

In accordance with yet a further disclosure, there is provided a method of producing a composition as herein above described, for use in the preparation of a medicament or food supplement, comprising:
a) fermenting *Lactobacillus plantarum* ECGC 13110402 under conditions sufficient to produce a culture broth;
b) concentrating the *Lactobacillus plantarum* from the culture broth so as to form a concentrate of the *Lactobacillus plantarum* cells;
c) subjecting the concentrate to a cryoprotectant so as to form a mixture;
d) freeze drying the mixture; and
e) adding one or more cholesterol modifying active ingredients to the mixture so as to form the composition.

The survival rates for freeze drying the *Lactobacillus plantarum* cells by such a method is over 70%. Furthermore, the method has been advantageously found that the method produces the *Lactobacillus plantarum* cells in amounts of up to 8 × 10¹¹ cfu/g.

### Detailed Description

Figure 1 is a graph showing the average *Lactobacillus* concentrations (±SD) in fecal batch culture (n=3) enumerated using the Lab158 FISH probe;
Figure 2A-2D are graphs showing that *L. plantarum* GOS enhances Bile Salt Hydrolase activity of *Lactobacillus plantarum* ECGC 13110402, where 2A shows the hydrolysis levels of Glycholate, 2B shows the hydrolysis levels of Glycodeoxycholate, 2C shows the hydrolysis of Taurocholate and 2D shows the hydrolysis of Taurodeoxycholate; and
Figure 3 is a graph showing the average cholesterol reduction (±SD) in faecal batch culture (n=3). *Lactobacillus plantarum* ECGC 13110402 significantly reduces cholesterol even in the absence of carbohydrate source. Effect is significantly enhanced in the presence of PGOS.

### Example 1 - Administration of Lactobacillus plantarum ECGC 13110402

A human volunteer study was conducted to establish the safety, compliance and extent of cholesterol reduction and hypertension control by administering compositions comprising *Lactobacillus plantarum* ECGC 13110402 to 49 mildly hypercholesterolaemic adults. The study was carried out independently by the Department of Food and Nutritional Sciences at the University of Reading, UK. The study was carried out according to the Helsinki declaration and written informed consent was obtained from all volunteers. The study protocol was approved by the Research Ethics committee of the University of Reading.

Subjects were male or female, aged 30 - 65 years. Subjects were excluded if they had had a previous cardiovascular event within the last 6 months, if secondary dyslipemias related to thyroid dysfunction were present, if they had used any drug affecting lipid metabolism in the previous 3 months, if they had a history of alcohol abuse, if they had taken antibiotics in the previous 6 months or if they had taken prebiotics/probiotic preparations in the last month.

Those who met the inclusion criteria were screened prior to the commencement of the study. A baseline blood sample was taken and their BMI and blood pressure were measured. The screening blood sample was analysed for full blood count (FBC) and liver function tests (LFTs) to determine overall health. Total cholesterol (TC), low density lipoprotein cholesterol (LDL-C), high density lipoprotein cholesterol (HDL-C), fasting triacylglycerol (TAG) and vitamin D were also measured. Urine, blood and faeces were collected for bile acid and metagenomic and metabolomics studies.

The study was a single-centre, prospective, randomized, double-blind, placebo-controlled, parallel-group trial. Subjects were randomly distributed into two groups: placebo or treatment with *Lactobacillus plantarum* ECGC 13110402. The placebo and treatment groups were provided with a blister packed DR 1 capsule. The treatment group received 120mg of active *Lactobacillus plantarum* ECGC 13110402 providing a dose of 1.8 × 10⁹ cells per capsule which was administered once or twice daily; once at breakfast and once in the evening as a dietary supplement. Participants were advised not to change their regular diet or physical activity throughout the trial period. Habitual diet was assessed by pre-validated 5-day food diaries (2 weekend and 3 week days).

Formulation details for the active and placebo formulations respectively are shown in tables 1 and 2 below:

**Table 1**

| **Ingredient** | **mg/capsule** | **Billion for capsule** | **g for production** |
|---|---|---|---|
| Probiotic powder | 120 | 8.4x10⁹ | 567.00 |
| Corn starch | 118.6 | | 560.39 |
| Magnesium stearate | 3.2 | | 15.12 |
| Silicon dioxide | 3.2 | | 15.12 |
| Capsule DR size 1 white | 75 | | 354.38 |
| TOTAL | 320 | | 1512 |

**Table 2**

| **Ingredient** | **mg/capsule** | **Billion for capsule** | **g for production** |
|---|---|---|---|
| Corn starch | 238.6 | | 1127.39 |
| Magnesium stearate | 3.2 | | 15.12 |
| Silicon dioxide | 3.2 | | 15.12 |
| Capsule DR size 1 white | 75 | | 354.38 |
| TOTAL | 320 | | 1512 |

Volunteers were pre-screened 2 weeks prior to the study start and were advised to refrain from any pre/probiotic intake. The study consisted of two phases: a treatment period (12 weeks) and a wash-out period (4 weeks). The study included a baseline visit at selection, a visit at the midpoint and at the endpoint of the treatment period (weeks 0, 6 and 12, respectively), and a fourth visit after the wash-out period (week 16).

An initial set of analyses examined the demographic and outcome variables at baseline to ensure that the two groups were well matched. Continuous variables were analysed using the unpaired t-test, whilst the Chi-square test was used for the categorical variables.

Study outcomes between the two study groups were analysed in terms of changes between timepoints. Four study periods were examined for changes in outcomes: baseline to midpoint (0-6 weeks), midpoint to endpoint (6-12 weeks), baseline to endpoint (0-12 weeks) and endpoint to washout (12-16 weeks). Data for each analysis was restricted to the particular two timepoints in the analysis. The analyses were performed using analysis of covariance (ANCOVA). The latter timepoint was used as the outcome variable, with the earlier timepoint considered as a covariate. This approach is mathematically preferable to simply comparing the change over time between groups, as it takes into account the variable starting values for the test and control group. The first set of analyses considered all study participants and different patient subgroups. These subgroups were based on baseline total cholesterol (<5mmol/l, 5-5.9 mmol/l and ≥60 mmol/l), gender and age (<50 yrs, 50-59 yrs, ≥60 yrs).

There were no safety, compliance, or tolerance issues reported by volunteers throughout the study. Three volunteers dropped out of the study due to antibiotic treatment for non-related illnesses which excluded them from further study participation.

Volunteers were asked to fill in daily gastrointestinal symptom diaries throughout the duration of the study and to report any adverse effects experienced. GI symptoms for abdominal pain, bloating and flatulence were recorded by volunteers as none (0), mild (1), moderate (2) or severe (3). As illustrated in Table 3 below, average scores of self-reported gastrointestinal (GI) symptoms from baseline to 12 weeks showed no significant difference in bowel movements per day or stool consistency (Bristol stool chart) between the groups. One volunteer in the active group reported moderate abdominal pain, bloating and flatulence, while in the placebo group two volunteers reported moderate flatulence. None of the study participants reported severe GI side effects during the baseline to 12 week treatment period and no significant differences in stool morphology and frequency were observed between treatment groups. All other volunteers reported no symptoms.

**Table 3**

| | Placebo | | Active | |
|---|---|---|---|---|
| | Average | SD | Average | SD |
| Bowel movements | 1.28 | 0.53 | 1.27 | 0.51 |
| Stool consistency | 3.35 | 1.25 | 3.55 | 0.90 |
| Abdominal pain | 0.15 | 0.18 | 0.32 | 0.47 |
| Bloating | 0.28 | 0.31 | 0.35 | 0.49 |
| Flatulence | 0.68 | 0.44 | 0.53 | 0.48 |

The baseline characteristics (total cholesterol, anthropometric measurements, systolic and diastolic pressure) were compared between the placebo (n=23) and active (n=23) groups and are shown in table 4 below. The results suggested no significant difference between the two study groups in terms of their demographics (age, sex) or for any of the lipid or anthropometric measures at baseline.

**Table 4**

| Variable | Placebo (n=23) | Active (n=23) | P-value |
|---|---|---|---|
| | Mean (SD) | Mean (SD) | |
| Age | 52.0 (8.4) | 52.3 (10.7) | 0.89 |
| Gender: Female | 14 (61%) | 18 (78%) | 0.20 |
| Gender: Male | 9 (39%) | 5 (22%) | |
| Total cholesterol | 5.22 (0.92) | 5.10 (0.71) | 0.62 |
| HDL cholesterol | 1.24 (0.31) | 1.40 (0.35) | 0.10 |
| LDL cholesterol | 3.44 (0.76) | 3.20 (0.68) | 0.28 |
| Triacylglycerides | 1.18 (0.45) | 1.11 (0.46 | 0.61 |
| Weight | 79.2 (16.5) | 72.1 (12.0) | 0.10 |
| BMI | 26.8 (5.0) | 26.7 (3.7) | 0.96 |
| Waist | 92.3 (13.5) | 89.6 (12.0) | 0.49 |
| Systolic BP | 118.7 (16.0) | 119.2 (13.2) | 0.73 |
| Diastolic BP | 71.0 (12.2) | 73.0 (8.0) | 0.52 |

Independent statistical analysis was performed to examine how the changes in lipid measurements over the course of the study varied between the two study groups. Changes between four pairs of time points (0-6 weeks; 0-12 weeks; 6-12 weeks; 12-16 weeks) were examined. Only analysis of data between baseline and end of treatment (0-12 week) is shown unless otherwise indicated. Clinically or statistically significant variations between other timepoints are highlighted.

Tables 5-7 below show the mean and standard deviation at baseline and the end of treatment at 12 weeks. Table 5 shows the changes in lipid measurements for all subjects (n=46) from baseline to 12 weeks. Table 6 shows the changes in lipid measurements for subjects with TC of <5mmol/l (n=23) from baseline to 12 weeks. Table 7 shows the changes in lipid measurements for subjects with TC of 5-5.9mmol/l (n=17) from baseline to 12 weeks. Analyses was carried out for 6.0+mmol/l but as this group only contained 6 subjects (3 active, 3 placebo) only statistically significant differences are reported, with appropriate caveats.

The group differences from the ANCOVA analyses are reported, with the mean difference and corresponding confidence interval. These are reported as outcome for active group minus outcome for placebo group adjusting for the baseline value. P-values indicating the significance of the results are reported.

TC, LDL-C, HDL-C and TAG concentrations are expressed in mmol/l.

The groups were stratified according to baseline cholesterol levels as shown in tables 5-7 and according to age (<50 years n=16; 50-59 years n=18; ≥60 years, n=12) and gender (female=32, male n=14).

**Table 5**

| **Outcome** | **Group** | **Baseline Mean (SD)** | **12 weeks Mean (SD)** | **Change Mean (SD) [range]** | **% Change Mean (SD) [range]** | **Group Difference Mean (95% Cl)** | **P-value** |
|---|---|---|---|---|---|---|---|
| **TC** | Placebo | 5.22 (0.92) | 5.33 (0.84) | 0.11 (0.66) [-1.0, 1.4] | 3.1 (13.4) [-14.6, 34.1] | 0 | 0.51 |
| | Active | 5.10 (0.71) | 5.12 (0.87) | 0.02 (0.56) [-1.3, 1.2] | 0.6 (10.5) [-22.4, 23.3] | -0.12 (-0.47, 0.24) | |
| | | 5.16 | | | | -2.3% | |
| **HDL** | Placebo | 1.24 (0.31) | 1.24 (0.29) | 0.00 (0.17) [-0.2, 0.5] | 1.5 (17.0) [-14.3, 62.5] | 0 | 0.23 |
| | Active | 1.40 (0.35) | 1.46 (0.42) | 0.06 (0.15) [-0.1, 0.5] | 3.4 (10.5) [-12.5, 33.3] | 0.06 (-0.04, 0.16) | |
| | | 1.32 | | | | +4.5% | |
| **LDL** | Placebo | 3.44 (0.76) | 3.54 (0.70) | 0.10 (0.62) [-0.9, 1.3] | 4.9 (19.5) [-22.9, 52.0] | 0 | 0.15 |
| | Active | 3.20 (0.69) | 3.13 (0.78) | -0.07 (0.53) [-1.3, 1.0] | -1.4 (15.6) [-36.4, 26.3] | -0.24 (-0.56, 0.09) | |
| | | 3.32 | | | | -7.2% | |
| **TAG** | Placebo | 1.18 (0.45) | 1.20 (0.39) | 0.03 (0.41) [-0.9, 0.6] | 10.9 (35.7) [-52.9. 83.3] | 0 | 0.96 |
| | Active | 1.11 (0.46) | 1.15 (0.65) | 0.04 (0.36) [-0.5, 0.8] | 3.6 (34.8) [-55.6, 114.3] | 0.01 (-0.22, 0.23) | |
| | | 1.14 | | | | + 0.8% | |

**Table 6**

| **Outcome** | **Group** | **Baseline Mean (SD)** | **12 weeks Mean (SD)** | **Change Mean (SD) [range]** | **% Change Mean (SD) [range]** | **Group Difference Mean (95% Cl)** | **P-value** |
|---|---|---|---|---|---|---|---|
| **TC** | Placebo | 4.50 (0.28) | 4.79 (0.43) | 0.29 (0.60) [-0.7, 1.4] | 7.1 (13.7) [-14.6, 34.1] | 0 | 0.31 |
| | Active | 4.53 (0.33) | 4.61 (0.42) | 0.08 (0.38) [-0.5, 1.0] | 2.0 (8.5) [-10.9, 23.3] | -0.19 (-0.56, 0.19) | |
| | | 4.51 | | | | -4.2% | |
| **HDL** | Placebo | 1.09 (0.28) | 1.11 (0.23) | 0.02 (0.19) [-0.2, 0.5] | 4.5 (22.3) [-14.3, 62.5] | 0 | 0.33 |
| | Active | 1.34 (0.30) | 1.44 (0.42) | 0.10 (0.19) [-0.1, 0.5] | 6.2 (12.9) [-12.5, 33.3] | 0.09 (-0.10, 0.27) | |
| | | 1.21 | | | | +7.4% | |
| **LDL** | Placebo | 2.88 (0.33) | 3.15 (0.47) | 0.26 (0.59) [-0.8, 1.3] | 10.5 (21.0) [-22.9, 52.0] | 0 | 0.03 |
| | Active | 2.71 (0.31) | 2.72 (0.28) | 0.02 (0.30) [-0.6, 0.6] | 1.3 (11.7) [-21.4, 26.1] | -0.39 (-0.74, -0.04) | |
| | | 2.79 | | | | -13.9% | |
| **TAG** | Placebo | 1.14 (0.51) | 1.15 (0.38) | 0.02 (0.41) [-0.9, 0.5] | 12.3 (37.3) [-52.9. 62.5] | 0 | 0.96 |
| | Active | 1.08 (0.39) | 0.97 (0.35) | -0.12 (0.25) [-0.5, 0.2] | -8.1 (25.4) [-55.6, 28.6] | -0.01 (-0.22, 0.23) | |
| | | 1.12 | | | | - 0.9% | |

**Table 7**

| **Outcome** | **Group** | **Baseline Mean (SD)** | **12 weeks Mean (SD)** | **Change Mean (SD) [range]** | **% Change Mean (SD) [range]** | **Group Difference Mean (95% Cl)** | **P-value** |
|---|---|---|---|---|---|---|---|
| **TC** | Placebo | 5.48 (0.25) | 5.52 (0.64) | 0.04 (0.74) [-0.8, 1.2] | 1.1 (13.8) [-14.5, 22.2] | 0 | 0.44 |
| | Active | 5.55 (0.24) | 5.25 (0.54) | -0.30 (0.64) [-1.3, 0.6] | -5.2 (11.4) [-22.4, 11.8] | -0.23 (-0.87, 0.40) | |
| | | 5.51 | | | | -4.17% | |
| **HDL** | Placebo | 1.30 (0.26) | 1.31 (0.33) | 0.01 (0.15) [-0.2, 0.2] | 0.3 (11.2) [-12.5, 15.4] | 0 | 0.91 |
| | Active | 1.50 (0.47) | 1.51 (0.49) | 0.01 (0.1) [-0.1, 0.2] | 0.6 (6.4) [-8.3, 11.8] | -0.01 (-0.15, 0.14) | |
| | | 1.4 | | | | -0.01 | |
| **LDL** | Placebo | 3.61 (0.27) | 3.63 (0.54) | 0.02 (0.67) [-0.8, 1.1] | 1.5 (19.2) [-21.1, 33.3] | 0 | 0.11 |
| | Active | 3.55 (0.40) | 3.16 (0.58) | -0.39 (0.65) [-1.3, 0.6] | -10.2 (18.3) [-36.4, 18.8] | -0.47 (-1.08, -0.13) | |
| | | 3.58 | | | | -13.1% | |
| **TAG** | Placebo | 1.24 (0.42) | 1.29 (0.48) | 0.04 (0.46) [-0.9, 0.6] | 10.7 (39.3) [-52.9. 83.3] | 0 | 0.56 |
| | Active | 1.11 (0.58) | 1.26 (0.92) | 0.15 (0.38) [-0.3, 0.7] | 5.9 (26.4) [-33.3, 41.2] | 0.13 (-0.33, 0.58) | |
| | | 1.17 | | | | +11.1% | |

Total cholesterol (TC) from baseline to 12 weeks was reduced in all groups compared to the placebo group (Tables 5-7). The baseline adjusted value for TC levels in all subjects was 0.12mmol/I lower in the active group compared to the placebo group, a 2.3% decrease. Stratification according to baseline TC concentrations revealed variations between the higher TC and medium to low subgroups. In the TC<5.0mmol/l subgroup the change between baseline and end of treatment was 4.2% lower in the active compared to the placebo group (0.19mmol/l lower). Similarly in the TC 5-5.9 mmol/l group, the baseline adjusted end of treatment TC concentrations were 0.23mmol/l lower in the active group, corresponding to a 4.17% reduction. In the TC≥6.0mmol/l (0-6 weeks) group a statistically significant reduction in TC of 2.44mmol/l was observed, corresponding to a 36.7% reduction (P=0.045) (data not shown in tables 5-7). However, the size of this group was very small (n=3 placebo/3 active) and so despite its statistical significance no group relevance should be attributed to this effect. No significant effect of gender on TC was identified. Although most results were not of statistical significance, there was a common group trend for a decrease in TC in the active treatment groups when compared to the placebo. The results also suggested that patients with higher initial levels of TC may benefit from higher reductions in TC than others.

HDL-C increased slightly between baseline and 12 weeks for both placebo and active groups. On adjusting for variable baselines the HDL-C concentrations for the all subject and TC <5mmol/l group were 0.06 mmol/l (4.5%) and 0.09mmol/l (7.4%) higher in the active group when compared to the placebo. Most of this difference occurred in the 6-12 week period where differences approaching statistical significance were seen for both all subject (p=0.06) and TC <5mmol/lgroups (p=0.09). The all subject and TC <5mmol/l groups in this period showed average increases in HDL cholesterol levels of 0.09mmol/l (6.5%) and 0.10mmol/l (7.8%) respectively when compared to the placebo group.

Age stratification revealed statistically significant group differences (p=0.03) in the 60+ group (n=12) who had average increases in HDL cholesterol of 0.23mmol/l (+14.7%) when compared to the placebo group. Stratification according to baseline TC concentrations and gender revealed no significant treatment effect on HDL levels.

LDL-C cholesterol reduced between baseline and 12 weeks in all the active treatment groups. This effect was not observed in the placebo group.

Upon adjusting for variable baselines, LDL-C concentrations for the all subject groups were on average 0.24mmol/l (7.2%) lower when compared to placebo. LDL-C concentrations in the TC<5.0mmol/l group were significantly lower by 0.39mmol/l (13.9%) in the active compared to the placebo group (P=0.03). These reductions have clinical and commercial significance. In the TC 5.0-5.9mmol/l group, LDL-C showed an average 0.47mmol/l decrease (13.1%), but this did not reach statistical significance. The LDL reducing effect appeared to occur consistently across both the 0-6 and 6-12 week periods. The results suggest patients with higher initial levels of LDL cholesterol may benefit from higher reductions in LDL-C than others.

Stratification according to gender revealed a more pronounced LDL-C reducing effect in female volunteers compared to males (p=0.06). Active group concentrations were 0.41mmol/l (12.4%) lower for females compared to placebo while a 0.06mmol/l (1.8%, P=0.06) increase was observed for the active male group compared to placebo (*P*=0.83).

Stratification according to age showed higher decreases in LDL-C concentrations with increasing age. Only slight changes were observed in the baseline adjusted LDL-C concentrations in the <50 years group (0.08mmol/l increase). LDL-C decreases were more pronounced in the 50-59 group (0.49mmol/l) and in the ≥60 years group (0.31mmol/l), corresponding to a 15% and 9.14% decrease respectively in the active group compared to the placebo.

No significant effects on triacylgyceride concentrations were observed upon the ingestion of either the active or placebo treatments in the all subjects, TC=<5mmol/l, or TC 5-5.9mmol/l groups. Age stratification showed a statistically significant (p=0.002) triglyceride reduction in the 60+ group of 0.48mmol/l (53.9%) between the placebo and active group. This large reduction in triglycerides was consistent across all testing time periods with a statistically significant reduction (p=0.03) of 0.26mmol/l (32.9%) in the 6-12 week period and a reduction of 0.28mmol/l(31.4%, p=0.07) in the baseline to 6 week period.

Changes in anthropometric measurements for all subjects (n=46) in the placebo and active treatment groups are shown from the baseline to the end of treatment after 12 weeks in table 8 below. The mean values and standard deviation for each measured outcome at baseline and after 12 weeks are shown in table 8. Group differences from the ANCOVA analyses are also shown with the mean difference and corresponding confidence interval. These are reported as outcome for active group minus outcome for placebo group adjusting for the baseline value. P-values indicating the significance of the results are reported. Body weight is expressed in kg, BMI in kg/m², waist circumference in cm and systolic/diastolic pressure in mmHg.

**Table 8**

| **Outcome** | **Group** | **Baseline Mean (SD)** | **12 weeks Mean (SD)** | **Change Mean (SD) [range]** | **% Change Mean (SD) [range]** | **Group Difference Mean (95% Cl)** | **P-value** |
|---|---|---|---|---|---|---|---|
| **Weight** | Placebo | 79.2 (16.5) | 79.3 (16.8) | 0.2 (1.7) [-2.6, 3.5] | 0.1 (2.1) [-3.3, 4.7] | 0 | 0.18 |
| | Active | 72.1 (12.0) | 72.8 (12.6) | 0.7 (1.7) [-2.6, 3.8] | 0.9 (2.2) [-2.8, 4.9] | 0.7 (-0.3, 1.7) | |
| **BMI** | Placebo | 26.8 (5.0) | 27.0 (5.2) | 0.3 (1.3) [-3.1, 4.2] | 0.9 (4.7) [-9.3, 15.5] | 0 | 0.41 |
| | Active | 26.7 (3.7) | 27.2 (4.0) | 0.5 (0.9) [-1.1, 3.3] | 2.0 (3.3) [-3.9, 11.8] | 0.3 (-0.4, 1.0) | |
| **Waist** | Placebo | 92.3 (13.5) | 90.5 (13.8) | -1.8 (6.4) [-14, 12] | -1.8 (6.8) [-17.3, 12.9] | 0 | |
| | Active | 89.6 (12.0) | 89.1 (11.0) | -0.5 (5.7) [-13, 13] | -0.2 (6.7) [-13.0, 16.3] | 0.9 (-2.6, 4.4) | 0.61 |
| **Systolic pressure** | Placebo | 117.7 (16.0) | 122.3 (11.4) | 4.7 (11.0) [-13, 28] | 4.9 (10.3) [-11.4, 31.1] | 0 | 0.15 |
| | Active | 119.2 (13.2) | 119.7 (13.0) | 0.5 (8.9) [-19, 21] | 0.7 (7.2) [-13.4, 15.8] | -3.6 (-8.6, 1.4) | |
| | | 118.45 | | | | -3% | |
| **Diastolic pressure** | Placebo | 71.0 (12.2) | 73.5 (8.2) | 2.4 (9.0) [-15, 18] | 5.0 (13.6) [-14.4, 30.5] | 0 | 0.39 |
| | Active | 73.0 (8.0) | 73.0 (8.2) | 0.0 (5.9) [-9, 13] | 0.3 (8.4) [-10.5, 20.3] | -1.6 (-5.2, 2.1) | |
| | | 72 | | | | -2.2% | |

No significant changes were noted in the anthropometric parameters relevant to weight, BMI and waist circumference between baseline and end of treatment at 12 weeks.

There was group evidence of a difference in systolic and diastolic blood pressure between baseline and 12 weeks. The difference in systolic blood pressure was both statistically and clinically significant. In the all subject active treatment group systolic blood pressure was 3.6mmHg lower (-3%) whilst diastolic pressure was reduced by 1.6mmHg (2.2%). The majority of the reduction in systolic blood pressure occurred in the 6-12 week time period. This showed a statistically significant reduction (*P*=0.003) in systolic blood pressure of 6mmHg (5.1%) in the active group when compared to the placebo group (data not shown in table 4). This is higher than the mean 3mmHg pulse pressure reduction achieved by ACE inhibitors, ARBs and renin inhibitors and the 2mmHg pulse pressure reduction with non-selective beta blockers. This reduction is also greater than the reduction of systolic BP by -3.56 mm Hg (95% confidence interval, -6.46 to -0.66) compared with control groups shown in a study analysing blood pressure reduction by probiotics (Khalesi et al, 2014).

The results show that *Lactobacillus plantarum ECGC* 13110402 has the potential to lower systolic blood pressure, blood TC and LDL-C in at least mildly hypercholesterolaemic subjects. These results, in an unoptimised product, suggest efficacy similar or greater to 1.5 - 2.4 g plant sterols/stanols per day.

Active *Lactobacillus plantarum* ECGC 13110402 and placebo capsules were stored at 4°C throughout the study duration. Product stability was checked at baseline, 6 weeks and 12 weeks (end of treatment) of the study and no significant change was observed in bacterial numbers. No bacterial growth was detected in the placebo capsules.

Analysis of safety parameters did not show deleterious effects of consuming *Lactobacillus plantarum* (ECGC 13110402). *Lactobacillus plantarum* is a widely used probiotic which is considered Generally Regarded as Safe (GRAS) by the US Food and Drug Administration (FDA) and has a Qualified Presumption of Safety (QPS) designation by the European Food Standard Agency. This would suggest that *Lactobacillus plantarum ECGC* 13110402 has the potential to be a safe and effective treatment for the treatment of hypercholesterolemia and hypertension.

Industrial scale-up experiments were also conducted on *Lactobacillus plantarum ECGC* 13110402. The following activities were performed: a) testing of flasks for different hypoallergenic media; b) fermentations of 1-5 L, concentration and freeze drying of small amounts to study; c) testing of different cryoprotectants; d) testing of different freeze drying curves; e) fermentation in 80 L, concentration and freeze drying. The final step was a production in a 80 L fermenter which resulted in: (i) cell count > 8 × 10¹¹ cfu/g; (ii) Aw: 0,11; (iii) a quantity of 700 g of concentrated biomass, freeze dried and not diluted/standardized with any excipient. Therefore, this particular strain looked extremely promising from a manufacturing point of view. Survival rate of the cells was found to be at more than 70% and yields were at 1.25% which is extremely high.

### Example 2 - The effect of purified Lactobacillus plantarum ECGC 13110402 GOS alone and in combination with Lactobacillus plantarum ECGC 13110402 on the human faecal microbiome

The effect of purified *L. plantarum* ECGC 13110402 GOS (LPGOS) alone and in combination with *L. plantarum* ECGC 13110402 (LP) on the human faecal microbiome was evaluated in anaerobic, pH and temperature - controlled, micro scale batch cultures in the presence of cholesterol. Purified, commercially available bifidobacterial GOS (BGOS) was used as a positive control.

Micro scale faecal batch cultures (10ml) testing in parallel: Faeces; Faceses + LP; Faeces + LPGOS; Faeces + LPGOS + LP; Faeces + BGOS; and Faeces + BGOS + LP.

Cultures (24h) were run in triplicate, using faeces from each of three healthy adults. Samples were obtained for bacteriology (FISH), metabolite, BSH activity and cholesterol reduction determination.

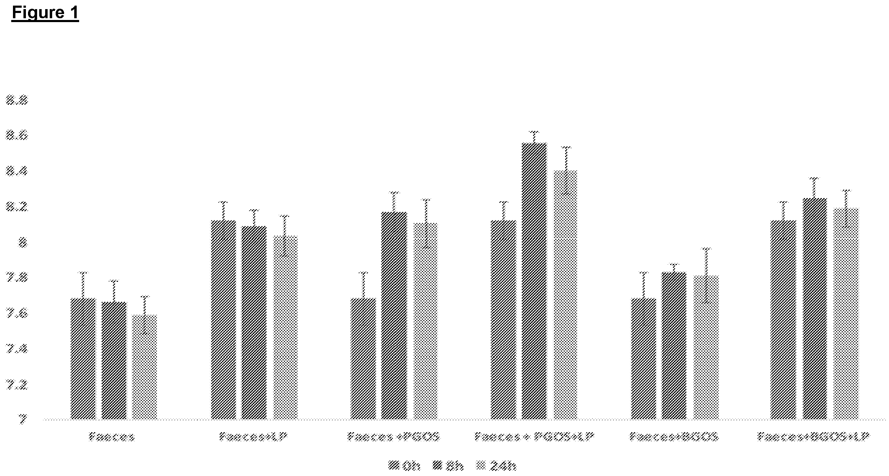

As illustrated in Figure 1, LPGOS significantly and selectively increased *Lactobacillus* populations, BSH and cholesterol reducing activities. The effect was further enhanced by combining LPGOS with *L. plantarum* ECGC 13110402.

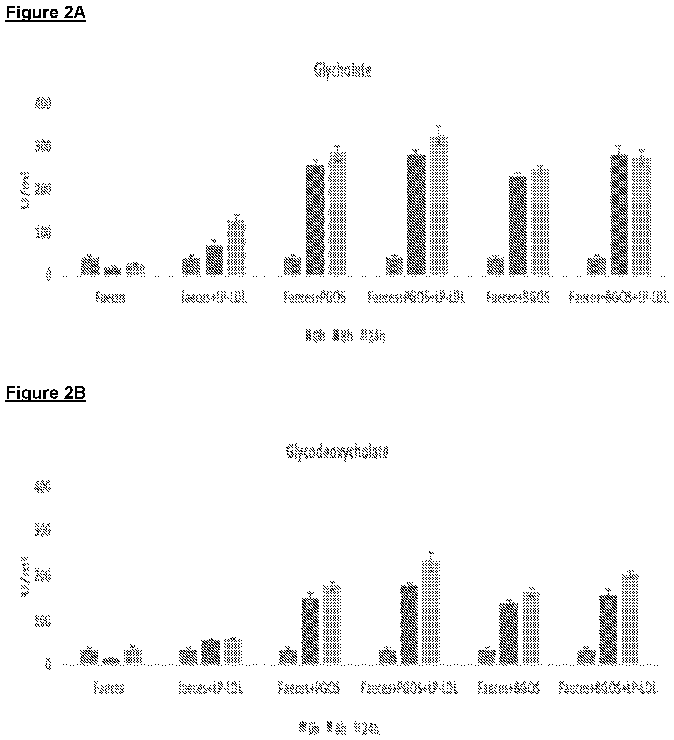

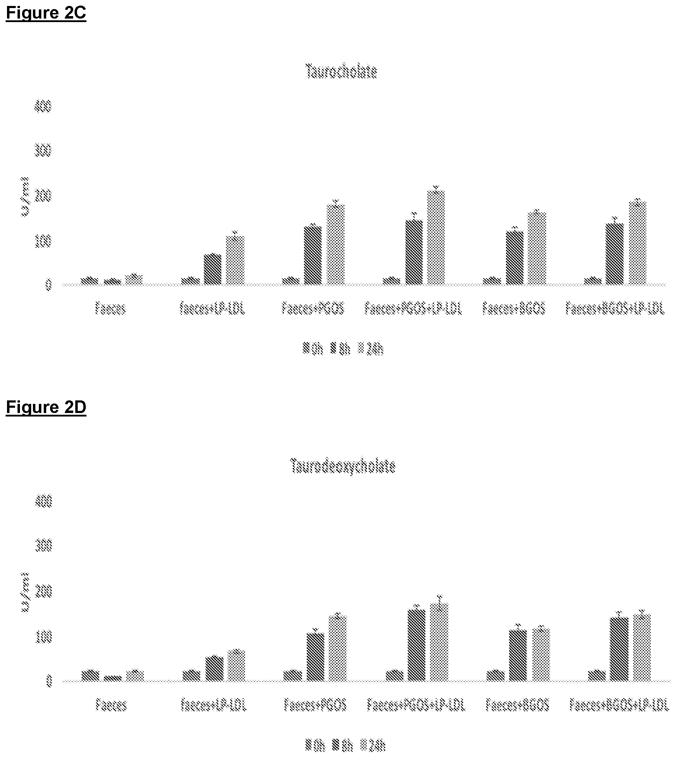

Figures 2A to 2D show that during the experiments, significantly higher glycholate and taurocholate hydrolysis by *L. plantarum* ECGC 13110402 was found when compared to faeces. PGOS and BGOS were found to significantly increase bile salt hydrolysis compared to faeces. Bile salt hydrolysis was found significantly higher for PGOS when used with L. *plantarum* ECGC 13110402 compared to BGOS combinations.

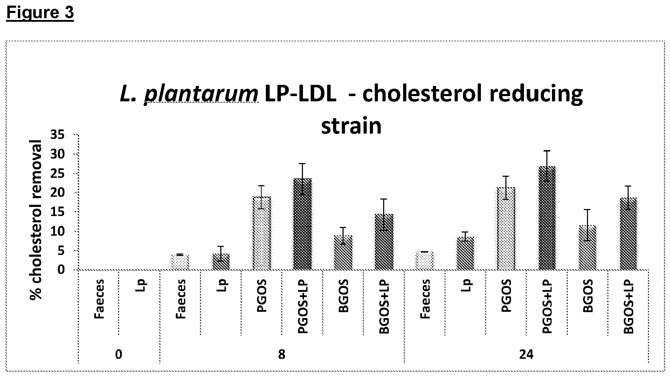

As illustrated in Figure 3, the effect on metabolic activity correlated with *Lactobacillus* concentrations indicating a true synergistic effect that was not observed when *L. plantarum* ECGC 13110402 was used in combination with BGOS. BGOS did not have an impact on cholesterol reduction.

In these experiments, it was found that using β-galactosidases expressed by *L. plantarum* ECGC 13110402 achieved the synthesis of GOS that works in true synergy with the parent strain, not only increasing its population but also impacting on the biological activity for which the probiotic was selected. The results of these experiments show that the presence of a prebiotic increases growth, increases BSH activity, and enhances cholesterol reduction by up to 3 times.

### Example 3 - Active ingredients

A number of active ingredients which have been shown to modify cholesterol may be combined with *Lactobacillus plantarum* ECGC 13110402.

### Inclusion criteria for active ingredients

Some of the active ingredients should be a food or a food derived ingredient for which there is evidence of well proven efficacy on the primary endpoint measure, serum LDL-cholesterol (LDL-C), from randomly controlled intervention trials in humans. Beneficial effects on secondary endpoints of cardio-metabolic risk, such as energy intake, glucose homeostasis, dyslipidaemia, blood pressure and vascular dysfunction, would be an advantage. The compound must be safe and tolerable for human consumption, and have a viable route of delivery to its site(s) of action (e.g. gut and/or peripheral tissues). In relation to effect on primary and secondary endpoints, its mechanism(s) of action should complement that of *Lactobacillus plantarum* ECGC 13110402.

Most of the compounds under address have an accepted health claim through European Food Safety Agency (EFSA), and comply with their criteria for the substantiation of a health claim in at least one case. The EFSA criteria is that the compound should benefit human health; there should be evidence of a cause and effect relationship on basis of the strength, consistency, specificity, dose-response and biological plausibility of the relationship. The quantity of the compound and its pattern of consumption required to obtain the claimed effect must be achievable within a balanced diet, and the specific study group in which the evidence was obtained must be representative of the target population for which the claim is intended.

### Background information of selected compounds

Some of the selected compounds can be categorised into non-digestible carbohydrates (dietary fibres), plant-derived polyphenols (phytosterols and stanols), and a polykelide (monocolin K). These compounds produce what is classified by the NICE guidelines as a *'low intensity reduction in serum LDL-C'* of between 20-30%. Their modes of action are mostly in the gut, and thus non-systemic, with the exception of monocolin K.

### Statins

Statins are a class of lipid-lowering medications that inhibit the enzyme HMG-CoA reductase and are used to control cholesterol levels in individuals. The National Institute for Health and Care Excellence (NICE) in the UK has provided guideline on dosage for satins. Statins are grouped into 3 different intensity categories according to the percentage reduction in low-density lipoprotein cholesterol:
- Low intensity (20-30% LDL-C reduction):
   ∘ fluvastatin 20-40 mg daily
   ∘ pravastatin 10-40 mg daily
   ∘ simvastatin 10 mg daily.
- Medium intensity (31-40% LDL-C reduction):
   ∘ atorvastatin 10 mg daily
   ∘ fluvastatin 80 mg daily
   ∘ rosuvastatin 5 mg daily
   ∘ simvastatin 20-40 mg daily.
- High intensity (more than 40% LDL-C reduction):
   ∘ atorvastatin 20-80 mg daily
   ∘ rosuvastatin 10-40 mg daily
   ∘ simvastatin 80 mg daily.

All of these medicaments have been clinically proven to reduce cholesterol.

### Plant/Phyto stanols and sterols

As food ingredients, phytosterols are esters of plant derived β-sitosterol, while phytostanols are esters of sitostanol and campestanol obtained from the reduction of the respective plant sterols from food grade plant oils (e.g. soybean). Both compounds have equivalent and well established efficacy in lowering serum LDL-C in mild hypercholesterolaemia, with an intake of 2-3g/d achieving a mean reduction in serum LDL-C of 7-10% in a matter of weeks to months in meta-anlyses. This effect has been demonstrated in a range of food matrices, including low fat products. There is some evidence of secondary health benefits on markers of inflammation, blood clotting and some cancers. The difference between phytosterols and stanols is that the former are absorbed (then re-secreted by efflux transporters in the enterocyte ABCG5 & G8) to a significantly greater extent than phytostanols (0.5-2% versus 0.04-0.2% respectively versus dietary and biliary cholesterol ∼55%). The LDL-lowering effect is mediated through two mechanisms; competition with dietary and biliary cholesterol for uptake and solubilisation into mixed micelles, and via the down-regulation of the Niemann Pick-C1-L1 transporter, both of which reduce the uptake of cholesterol into the enterocyte. This non-systemic reduction in the absorption of dietary (0-0.5g/d) and biliary cholesterol (~1g/d) limits cholesterol supply, chiefly to the liver, depleting intra-cellular free cholesterol. This promotes a compensatory up-regulation of LDL-receptor gene expression and activity, and the removal of circulating LDL into cells. Health claims and recommendations - EFSA states that plant sterols and stanols *'help in the management of normal cholesterol levels'*(ID 549, 550, 567, 713, 1234, 1235, 1466, 1634, 1984, 2909, 3140), and that a cause and effect relationship has been established between these compounds and the lowering of serum LDL-C. ATP-III guidelines in the USA state that phytosterols and stanols should be recommended for the long term management of hypercholesterolaemia, and as a useful adjunct to statin therapy in the reduction of CVD risk. Safety and tolerance - The main safety issue concerns a reduction in the absorption of fat soluble vitamins, and for this reason phytosterols and stanols are contraindicated in pregnant and breast feeding women, and children under 5 years. They are also contraindicated for individuals with a rare inherited condition of sitosterolaemia who have a mutation in their cholesterol efflux transporters (ABCG5/G8) and thus an inability to re-secrete absorbed plant sterols which leads to their accumulation in cells and the artery wall. There is limited evidence to suggest that small numbers of individuals without this rare condition may also accumulate sterol esters and are thus at increased CVD risk.

A review of stanols and sterols by the EFSA concluded that free (unesterified) stanols and sterols at doses of 1.5 g - 1.9 g and 2.0 g - 2.4 g per day lowered blood LDL-cholesterol by an average of 8.5 % and 8.9 % respectively (The EFSA Journal (2009) 1175, 1-9). However, studies (Katan MB, et. al., (2003) Mayo. Clin. Proc. 78, 965-978) have also showed that lower doses of stanols and sterols at 0.7 g - 1.1 g per day reduced LDL-cholesterol by 6.7 % and higher doses at over 2.5 g per day reduced LDL-cholesterol by 11.3 %.

Phytosterols (added as free or esterified sterols) have been incorporated into appropriate foods such as milk and yoghurt. Plant stanol esters have also been added to foods (mainly margarine type spreads but also including yoghurt, mayonnaise, gel capsules, butter, low fat cheese, milk, muesli, "ready-made low fat meals", and pasta)

### Monacolin K in red yeast rice

Stains are the first line treatment for the management of hypercholesterolaemia, but suffer from a poor safety record and low tolerance from common side effects (e.g. myalgia). As a results, the long term adherence to statins can be as low as 40% Alternatively, fermented red yeast rice (RYR) is a traditional Chinese food that been used as a relatively safe dietary supplement and herbal medicine for centuries and contains as its active ingredient, monocolin A which is identical in structure to one of the first generation of statins, lovastatin. RYR is has been reported to have the same efficacy in lower LDL-C as simvastatin and pravastatin. While this finding has not been reproduced for RYR at the high doses at which these statins are administered (20-50mg/d), at 10mg/d, RYR has been shown to lower LDL-C by more than of 15-20% in randomly controlled trials. Health claims - EFSA (ID 1648, 1700) health claims associated *'cholesterol management and heart health'* and specifically that monocolin K from RYR *'contributes to the management of normal blood cholesterol levels'.* Moreover, a cause and effect relationship has been established for an effect on LDL-C in the general population. Safety - There have been numerous reports of adverse side effects to monocolin K at intakes as low as 5mg/d, and safety warnings issued.

A review of monacolin K from red yeast rice (rice fermented with the red yeast *Monascus purpureus*) by the EFSA concluded that there was a relationship between the consumption of monacolin K from red yeast rice and maintenance of normal blood LDL-cholesterol concentrations (The EFSA Journal (2011), 9, (7), 2304*).*

The EFSA considered that in order to obtain to maintain normal blood LDL-cholesterol concentrations, 10 mg of monacolin K from fermented red yeast rice preparations should be consumed daily. Various red yeast rice preparations are available as food supplements. The preparations from red yeast rice typically contain starch, protein, fat (including monounsaturated fatty acids, plant sterols), isoflavones, and other compounds. Depending on the *Monascus* strains used and the fermentation conditions, the products may contain polyketides called monacolins, which are secondary metabolites produced during fermentation (Liu, J., et. al., (2006) Chinese Medicine, 1, 4)*.*

Monacolin K, in lactone (also known as lovastatin or mevinolin) and hydroxy acid forms, is the main monacolin in *Monascus purpureus*-fermented rice (75-90 % of total monacolin content) (Heber D., et. al., (2004) Am. J. Clin. Nutrition, 69, 231-236; *and* Li, Y.G., et. al., (2004) J. Pharm. Biomed. Analysis, 35, 1101-1112)*.* Commercially available red yeast rice products have variable contents of monacolin K and total monacolins. A dose of 7.5 mg/day over a 12 week period has been shown to reduce LDL-cholesterol concentrations by 16% from baseline. Other studies which used a dose of 2.5 mg/day over a 8 week period also showed LDL-cholesterol concentrations reductions.

### Soya Protein

Soya protein with or without soya isoflavones have been found to reduce cholesterol *(*Harland, J.I., et. al., (2012) Nutritional Research Reviews, 25, (2), 249-266). A daily intake of 25g soya protein has been shown to provide an equivalent to 5.5% reduction in LDL-cholesterol. Substitution of 13-58g soya protein containing foods per day for animal protein foods showed a 3.6 - 6.0% reduction in LDL-cholesterol. Currently, health claims in Europe and USA are allowed for the intake of 25 g soya to be associated with lowered blood cholesterol.

### Dietary fibres

There is strong and incontrovertible evidence for the beneficial effects of dietary fibre in reducing the risk of chronic degenerative diseases (cardiovascular disease, diabetes and certain cancers), and a critical need for populations at risk of these diseases to meet dietary recommendations for intake (e.g. UK 30g/d from current national average intake of ~12g/d adults, NDNS 2014). Dietary fibre is non-digestible carbohydrates in whole grains, fruits vegetables and legumes that are classified by their water solubility, viscosity, fermentation and functional properties. The selection of fibres below is primarily based on the existing and emerging weight of evidence for their efficacy in reducing serum LDL-C, and existence of accepted health claims.

### Soluble Fibre

### β-Glucans

β-Glucan is a soluble, highly viscous and fermentable dietary fibre composed of linear chains of glucose, that is derived chiefly from oats and barley. While intakes of between 2-10g/d have been consistently associated with reductions in serum LDL-C in meta-analyses, an intake of 3g/d has been identified as a critical threshold for achieving a reduction in LDL-C of ~0.3mmol/l (~10%), above which there is no further effect. The efficacy of the LDL-C lowering is greater in hypercholesterolaemic individuals, and in forms of β-glucans with higher molecular weight and viscosity. There is some evidence to suggest that β-glucans also lowers serum glucose and raises HDL-C. The mechanism of action of β-glucans on LDL-C is primarily via the binding, excretion and thus further hepatic secretion, of bile acids. This interruption of the entero-hepatic circulation of bile acids depletes the hepatic pool of free cholesterol, which in turn up-regulates the gene expression and activity of LDL receptors. Health claims - EFSA *'Regular consumption of β-glucans contributes to the maintenance of normal blood cholesterol concentrations'* (ID 1236, 1299); *may increase satiety leading to a reduction in energy intake* (ID 851, 852); *may lower postprandial glycaemic response* (ID 821, 824). Cause and effect relationships have been established for the former and latter claims. The U.S. Federal Regulations state that 3g/d or more from either whole oats or barley or in combination can reduce risk of coronary heart disease.

3g of soluble fibre from oats (3 servings of oatmeal, 28g each) has also been found to decrease LDL-cholesterol by approximately 0.13 mmol/l (Brown L., et. al., (1999) Am. J. Clin. Nutr. 69, 30-42)*.* Barley β-glucans has also been shown to have cholesterol lowing properties. By reference to a sub-analysis it was established that 3 g barley β-glucan was the minimum effective dose and, at this level of inclusion, LDL-cholesterol is reduced by 0.28 mmol/l, a reduction of approximately 7% compared with baseline, and total cholesterol by 0 34 mmol/l, a reduction of 5 7% (*EFSA Report (2010) EFSA J, 8, 1885*). In order to carry the health claim in Europe, foods must meet the condition that 3 g β-glucans per day from oats, oat bran, barley, barley bran, or from mixtures of non-processed or minimally processed β-glucans, should be contained in one or more servings. To achieve this level of β-glucans intake, a typical serving of cereals required is about 84 g per day.

### Pysllium

A soluble, viscous (gel-forming), non-fermented dietary fibre derived from the wheat husk of the seed of the Plantango plant, that is used as a bulk-forming laxative to treat constipation. Its metabolically active component is arabinoxylan. Intakes of 5-15g/d have been reported to lower serum LDL-C by between 5-12%. It has also been reported to lower serum triglycerides, elevate HDL-C, and improve glycaemic control, especially in type 2 diabetes. Its mechanism of action may be through its gel-forming properties that increase the viscosity of chyme, reduce contact with digestive enzymes, thus delaying the absorption of nutrients. The delivery of increased amounts of carbohydrate to the ilieum may also elevate GLP-1, which is known to improve glycaemic control and reduce energy intake by reducing appetite and increasing satiety. Health claims - EFSA 'Inclusion of this fibre in a healthy diet and lifestyle may lead to a reduction in blood cholesterol' (ID 4330). The U.S. federal regulations state that 7g or more per day of fibre from psyllium seed husk can reduce the risk of CHD. Safety - can cause gas and stomach cramps and must be ingested with water to avoid swelling in the throat and choking.

### Glucomannan

A water soluble, highly viscous type of fibre that does not occur naturally in food, but is extracted from the tubers of the *Konjac* plant, and is used as an emulsifying agent and thickener in foods. There is evidence to suggest that it may help to promote weight loss in overweight and obese individuals, and produce favourable effects on several biomarkers of cardio-metabolic health, including serum LDL-C. Much of the evidence for its beneficial effects are from animal studies (rat mainly). Data from randomly controlled trials in humans support a modest effect on weight loss, but there are no long term studies (>3 months). Its effects on biomarkers of risk are inconclusive. Health claims - EFSA *'Contributes to the reduction of body weight in the context of an energy-restricted diet'* (3g/d); '*Helps to maintain physiological lipid levels (cholesterol and triglyceride) ...and heart health'.* There is sufficient evidence for a cause and effect relationship between glucomannan and body weight, but not for the claims on blood lipids. The latter also applies to numerous other claims. Safety - there is no safety data in 'at risk' groups. Glucomannan may interfere with normal glucose homeostasis and has physical side effects that include intestinal blockage.

### Pectin

Viscous fibre in the form of pectins has also been recognised blood cholesterol lowering effects (*Brown (1999)*). Statistically significant effect of pectins on total and LDL-cholesterol at intakes of 2.2 - 9 g per day has been reported. There was also a significant dose-response relationship between the intake of soluble fibre (including pectins) and total and LDL-cholesterol lowering. It was estimated that 1 g pectins per day produced significant changes in total and LDL-cholesterol of 20.07 (95% CI 20.117, 20.022) and 20.05 (95% Cl 20.087, 20.022) mmol/l, respectively (both P,0.05). It has been suggested that in order to make a claim relating to the cholesterol effect, foods should provide at least 6 g of pectins per day in one or more servings (The EFSA Journal (2006) 8, 1747)*.*

### Chitosan

Another polysaccharide component for which an association between the maintenance of LDL-cholesterol and its consumption exists is chitosan (The EFSA Journal (2011) 9, 2214). The EFSA assessed evidence relating this polymer of β-1-4-linked D-glucosamine and N-acetyl-D-glucosamine, which is a component of the exoskeleton of crustaceans and the cell walls of
fungi. They suggested that the evidence indicated a small, but statistically significant effect on the reduction of both total (combining five studies) and LDL-cholesterol (combining two studies) concentrations, with no effect observed on HDL-cholesterol. The Panel suggested that in order to have an effect on blood lipids, 3 g chitosan per day should be consumed. The mechanism by which chitosan is presumed to exert the claimed effect is far from conclusive, but it was suggested that it binds to negatively charged lipids and reduces their gastrointestinal uptake.

### Tree Nuts

In a systematic review of studies that examined the relationship between the intake of nuts and their effect on blood lipids, it was found that in three almond (intake 50-100 g per day), two groundnut (35-68 g per day), one pecan nut (72 g per day) and four walnut (40-84 g per day) studies that the decreases in total cholesterol were between 2 and 16% and those in LDL-cholesterol were between 2 and 19 %, compared with subjects consuming control diets (Mukuddem-Petersen J., et. al., (2005) J Nutr 135, 2082-2089)*.*

A further meta-analysis of five RCT (randomised control trials) (n 142) conducted specifically with almonds, where intake was 25-168 g per day, demonstrated a reduction in total cholesterol of 0.18 (95% Cl 20.34, 20.02) mmol/l (P,0.05) and LDL-cholesterol of 0.15 (95% Cl 20.29, 0.00) mmol/l (NS). Studies conducted with walnuts were reviewed and random-effects meta-analysis of blood lipid outcomes was conducted in thirteen RCT (n 365). In these RCT, walnuts provided 10-24% of total energy intake. Diets supplemented with walnuts resulted in a significant reduction in total and LDL-cholesterol of 0.27 and 0.24 mmol/l, respectively (both P , 0.001).

In 2003 in the USA, a Qualified Health Claim was approved, which identified that the data suggested, that the intake of 1.5 oz (42 g) nuts per day may reduce the risk of heart disease.

### Flavonoids

There has been some evidence recently that flavonoids, which are found in food products such as green tea and chocolate can lower cholesterol.

### Garlic

There has been some evidence recently that garlic can lower cholesterol.

### Prebiotics

### Inulin

A 3-week study found that daily intake of 20 g of inulin significantly (*P* < 0.05) reduced serum triglycerides in 8 healthy volunteers (23-32 years old, BMI of 19-25 kgm⁻²). Similarly, a study involving eight healthy volunteers with a daily consumption of 10 g of inulin for three weeks also reached similar conclusions with a reduction of triglycerides in the region of 16.3% (Letexier, D., et. al., (2003) Am. J. Clin.Nutr. 2003, 77, 559-564). In animal studies, 10 male golden Syrian hamsters, mean BW of 58 ± 4 g were given 16% of inulin daily for 5 weeks which resulted in TC: 29% decrease (*P* < 0.05) and TG: 63% decrease (*P* < 0.05) (Trautwein, E.A., et. al, (1998) J. Nutr. 128, 1937-1943).

Other indigestible and fermentable compounds such as germinated barley, oligodextrans, gluconic acid, lactose, glutamine, hemicellulose-rich substrates, resistant starch and its derivatives, lactoferrin derived peptide, and *N*-acetylchitooligosaccharides have also been identified to have prebiotic potential with hypocholesterolemic effects.

### Fructooligosaccharides (FOS)

Twenty diabetic & hypercholesterolemic volunteers with fasting serum TC concentrations > 6 mmol/L were given 15 g per day, two 20 days treatment periods, with no washout period between treatments. HDL-C was found to increase by 2.8% (Alles, M.S., et. al. (1999) Am. J. Clin. Nutr. 69, 64-69).

### Galactooligosaccharides (GOS)

Forty four elderly volunteers (16 men & 28 women between 64-79 years old) were given 5.5 g per day of GOS, two 10 weeks treatment periods with a 4-week washout period *(*Vulevic, J., et. al., (2008) Am. J. Clin. Nutr. 88, 1438-1446). Whilst no significant improvements in lipid profiles were identified, it is believed that GOS may still confer a hypocholesterolemic effect at the right concentration.

### Chitooligosaccharides (COS)

Forty Nine male Arbor Acres broiler chickens (196 days old) were given 100 mg/kg BW

Daily for 42 days. This resulted in TG: 26.9% decrease (*P* < 0.05) and HDL-C: 12.3% increase (*P* < 0.05) (Li, X.J., et. al., (2007) Poultry Sci. 86, 1107-1114)*.*

### Xylooligosaccharides (XOS)

Forty male Sprague-Dawley rats (6 weeks old) were given a 60 g XOS/kg diet for 35 days. This resulted in TG: 33.9% decrease (*P* < 0.05) (Hsu, C.K., et. al., (2004) J. Nutr. 134, 1523-1528).

### Soybean Oligosaccharides

Fifty Wistar rats (4-weeks old) were hypercholesterolemic induced and given a diet of 450 mg/kg BW /day for 45 days. This resulted in TC: 38.5% decrease (*P* < 0.05) LDL-C: 43.0% decrease (*P* < 0.05) TG: 40.8% decrease (*P* < 0.05) HDL-C: 81.9% increase (*P* < 0.05) (compared to the positive control group) (Chen, H., et. al., (2010) Food Chem. 119, 1633-1636).

### Fatty Acids

### Alpha-linolenic acid (ALA)

A review of ALA by the EFSA concluded that there was a relationship between the dietary intake of ALA and the reduction of blood cholesterol concentrations (The EFSA Journal (2009), 7, (9), 1252). In order to bear a functional claims, a food should contain at least 15% of the proposed labelling reference intake value of 2 g ALA per day.

Indeed, clinical trials comparing the effects of different vegetable oils on serum lipids in normolipidaemic subjects have shown that the effect of alpha-linolenic acid (ALA) on serum cholesterol is similar to that of linoleic acid (LA)

A study looking at 3.4 g/day of ALA (by way of using flax oil and rice oil in bread rolls), resulted in systolic BP being significantly lower (about - 4 mmHg) after 4, 8 and 12 weeks *(*Takeuchi H., et. al., (2007) J. Oleo. Sci. 56, 347-360)*.* A 12-week study compared the effect of daily supplementation with flaxseed oil (15 ml providing 8 g ALA and 2 g LA, n=59) with safflower oil (15 ml providing 0.1 g ALA and 11 g LA, n=28) on BP in middle-aged dyslipidaemic men. Background diets of the two groups had similar quantities of ALA (1g/d) and total PUFA (12 g/d). After 12 weeks, a median decrease of 3% in systolic BP and of 6% in diastolic BP was observed (median decrease around -5 mm Hg) in the intervention group compared to controls (Paschos G.K., et. al., (2007) Eur. J. Clin. Nutr. 61, 1201-1206)*.*

ALA can be found in approximately 2-3 g flaxseed and can be provided either whole or as its lignin component.

### Long chain n-3 polyunsaturated fatty acids (PUFA)

Dietary long chain n-3 PUFA, eicosapentaenoic and docosahexaenoic acids (EPA, DHA or 'omega-3s'), derived chiefly from oily fish, are associated with reduced CVD risk and the prevention of sudden cardiac death, in part, through their anti-arrhythmic effects and ability to stabilise atherosclerotic plaque. These fatty acids also exert favourable physiological effects on cardio-metabolic risk factors, such as improving vascular dysfunction, lowering blood pressure, modulating inflammatory and immune function and reducing serum triglyceride in the post-prandial and post-absorptive (fasted) states. The latter improves lipoprotein profile, chiefly by increasing the capacity to hydrolyse TG-rich lipoproteins and lower the serum concentration of atherogenic lipoprotein remnants, and by doing so, reducing the propensity to deposit visceral fat, and ectopic fat in the liver. One potentially adverse effect of EPA/DHA is to increase serum LDL-C by between 5-10%. However, while raised serum LDL-C can co-exist alongside cardio-metabolic risk factors in insulin resistant conditions of obesity, metabolic syndrome and type-2 diabetes (cholesterol biosynthesis in the body increases with weight gain), it is not strictly a 'cardio-metabolic' risk factor. Cardio-metabolic risk is characterised by a predominance of small, dense LDL (sdLDL) which are relatively depleted of cholesterol, and express a reduced affinity for the LDL receptor and increased atherogenic potential. One favourable effect of the hypo-triglyceridaemic action of EPA/DHA is to remodel sdLDL into large LDL particles. This results in a small increase serum LDL-C, but at the same time renders LDL more receptor active and less atherogenic.

In practice, this effect can be exploited by combining EPA/DHA with an LDL-C lowering agent that works by up-regulating LDL receptor activity, either directly (e.g. statin) or indirectly by reducing cholesterol absorption in the gut (e.g. sterol/stanol or a pro-biotic). These complementary mechanisms of action help to explain why the addition of fish-oil increases the efficacy of statins in lowering serum LDL-C.

High-fish diets (1.23 g/day eicosapentaenoic acid (EPA) and docosahexaenoic acid (DHA)) are known to decrease non-fasting plasma triglycerides, with concomitant reductions in triglyceride-rich lipoprotein (TRL) apoB-100 concentration and production rate, compared with a low-fish diet in elderly men and women with moderate hyperlipidemia (Ooi, E.M., et. al., (2012) J. Lipid Res. 53, 1958-1967). The study was a 12-week, randomized intervention trial of a hypocaloric diet (energy deficit of ≥1900 kJ) and n-3 PUFA supplement (4 g/day Omacor; 46% EPA and 38% DHA) compared with a hypocaloric diet alone on TRL apoB-48 metabolism in obese men and women following ingestion of an oral fat load (a total of 4800 kJ, 130 g fat, 17 g protein, and 21 g carbohydrate). Studies have shown that weight loss and n-3 PUFA supplement decreased fasting and total postprandial TRL apoB-48 area under the curve (AUC) compared with weight loss alone.

### Omega-6 Polyunsaturated Fatty Acids (n6-PUFAs)

A recent study by van Schalkwijk *et al.* examined the effect of 60 g per day of dietary *n*-6 PUFA (71% linoleic acid) compared with medium-chain fatty acid (MCFA; 69% C8:0 and C10:0) supplementation on fasting lipoprotein profile and metabolism in 12 overweight-obese men using a randomized, double-blind crossover study design (Van Schalkwijk, D.B., et al., (2014) PLoS ONE, 9, e100376*).* Three weeks supplementation with n-6 PUFA lowered fasting total, very low-density lipoprotein (VLDL) and LDL cholesterol, and total plasma, VLDL and LDL triglyceride concentrations compared with MCFA.

### Example 4 - Combinations

The following formulations exemplify daily doses of the combinations of *Lactobacillus plantarum* ECGC 13110402 and one or more of the above active ingredients in products currently undergoing development, formulation and trials.

### Combination 1 - L. Plantarum, Monacolin K and Vitamin B₃ (Niacin)

This combination is for the maintenance of healthy cholesterol:
- *Lactobacillus plantarum* ECGC 13110402 (5 Billion cells), a natural and proprietary microbiome-modulator strain identified by the present inventors with clinically-proven efficacy to regulate the metabolism of bile acids from the liver, reducing their reabsorption in the intestine.
- Monacolin K from red yeast rice (10 mg), which contributes to the maintenance of normal blood cholesterol levels by inhibiting the synthesis of cholesterol by the liver. It is a natural product obtained by the fermentation of rice by the yeast *Monascus purpureus* which is used in China as a traditional remedy for high cholesterol.
- Vitamin B₃ (Niacin) (16 mg), which contributes to a normal energy-yielding metabolism by modulating the degradation of fats in the cell.

### Combination 2 - L. Plantarum, Plant stanols

This combination is for the maintenance of healthy cholesterol:
- *Lactobacillus plantarum* ECGC 13110402 (2.5 Billion cells), a natural and proprietary microbiome-modulator strain identified by the present inventors with clinically-proven efficacy to regulate the metabolism of bile acids from the liver, reducing their reabsorption in the intestine^{.}
- Plant stanols (400 mg) which contribute to the maintenance of normal blood cholesterol levels by competing with dietary cholesterol for absorption in the intestine. Plant stanols are virtually unabsorbable, which makes them more ideal hypocholesterolaemic agents than plant sterols^{.}

### Combination 3 - L. Plantarum, Omega-3 fatty acids EPA and DHA

This combination is for the maintenance of overall cardiovascular and heart health:
- *Lactobacillus plantarum* ECGC 13110402 (5 Billion cells), a natural and proprietary microbiome-modulator strain identified by the present inventors with clinically-proven efficacy to regulate the metabolism of bile acids from the liver, reducing their reabsorption in the intestine
- Omega-3 fatty acids EPA and DHA (250 mg), which help maintain the normal function of the heart by providing the body with essential triglycerides and keeping healthy blood vessels. Furthermore, a daily intake of 2 to 3 g of EPA/DHA support normal blood pressure and triglyceride levels

### Combination 4- L. Plantarum, Vitamin B₁ (Thiamine)

This combination is for the maintenance of overall cardiovascular and heart health:
- *Lactobacillus plantarum* ECGC 13110402 (5 Billion cells), a natural and proprietary microbiome-modulator strain identified by the present inventors with clinically-proven efficacy to regulate the metabolism of bile acids from the liver, reducing their reabsorption in the intestine
- Vitamin B₁ (Thiamine) (1 mg), which contributes to the normal function of the heart² by enabling the normal functioning of the heart muscle

### Combination 5 - L. Plantarum, Potassium

This combination is for the maintenance of healthy blood pressure:
- *Lactobacillus plantarum* ECGC 13110402 (2.5 Billion cells), a natural and proprietary microbiome-modulator strain identified by the present inventors with clinically-proven efficacy to regulate the metabolism of bile acids from the liver, reducing their reabsorption in the intestine. Microbiome-derived bile acid metabolites have shown to be able to reduce the vascular constriction caused by stress hormones and relax the blood vessels
- Potassium (150 mg), which contributes to the maintenance of normal blood pressure by modulating the metabolism of sodium and the activity of blood vessels

### Combination 6 - L. plantarum, Monacolin K, Potassium, Chromium

This combination is for a healthy ageing of the metabolism:
- *Lactobacillus plantarum* ECGC 13110402 (2.5 Billion cells), a natural and proprietary microbiome-modulator strain identified by the present inventors with clinically-proven efficacy to regulate the metabolism of bile acids from the liver, reducing their reabsorption in the intestine. Microbiome-derived bile acid metabolites have shown to be able to reduce the vascular constriction caused by stress hormones and relax the blood vessels.
- Monacolin K from red yeast rice (5 mg), which contributes to the maintenance of normal blood cholesterol levels by inhibiting the synthesis of cholesterol by the liver. It is a natural product obtained by the fermentation of rice by the yeast *Monascus purpureus* which is used in China as a traditional remedy for high cholesterol.
- Potassium (150 mg), which contributes to the maintenance of normal blood pressure by modulating the metabolism of sodium and the activity of blood vessels.
- Chromium (20 µg), which contributes to the maintenance of normal blood glucose levels and to the overall normal macronutrient metabolism by mediating in the metabolism of glucose and lowering physiological insulin resistance. Chromium The term "chromium" covers all dietary chromium salts, including trivalent chromium (Cr(III) or Cr³⁺) which naturally occurs in trace amounts in foods and waters. Preferably, the chromium salt comprises chromium picolinate.

### Example 5 - Worked Examples

The following formulations exemplify daily doses of the combinations of *Lactobacillus plantarum* ECGC 13110402 and one or more of the above active ingredients.

### Formulation A

| Ingredient | Amount |
|---|---|
| *Lactobacillus plantarum* ECGC 13110402 | 240 mg providing |
| | 2.6 × 10⁹ cells |
| simvastin | 80 mg |

### Formulation B

| Ingredient | Amount |
|---|---|
| *Lactobacillus plantarum* ECGC 13110402 | 240 mg providing |
| | 2.6 × 10⁹ cells |
| simvastatin | 10 mg |

### Formulation C

| Ingredient | Amount |
|---|---|
| *Lactobacillus plantarum* ECGC 13110402 | 240 mg providing |
| | 2.6 × 10⁹ cells |
| simvastin | 7.5 mg |

### Formulation D

| Ingredient | Amount |
|---|---|
| *Lactobacillus plantarum* ECGC 13110402 | 240 mg providing |
| | 2.6 × 10⁹ cells |
| Pytosterols | 2.5 g |

### Formulation E

| Ingredient | Amount |
|---|---|
| *Lactobacillus plantarum* ECGC 13110402 | 240 mg providing |
| | 2.6 × 10⁹ cells |
| Pytosterols | 0.7 g |

### Formulation F

| Ingredient | Amount |
|---|---|
| *Lactobacillus plantarum* ECGC 13110402 | 240 mg providing 2.6 × 10⁹ cells |
| Pytosterols | 0.5g |

### Formulation G

| Ingredient | Amount |
|---|---|
| *Lactobacillus plantarum* ECGC 13110402 | 240 mg providing 2.6 × 10⁹ cells |
| Monacolin K | 10 mg |

### Formulation H

| Ingredient | Amount |
|---|---|
| *Lactobacillus plantarum* ECGC 13110402 | 240 mg providing 2.6 × 10⁹ cells |
| Monacolin K | 2.5 mg |

### Formulation I

| Ingredient | Amount |
|---|---|
| *Lactobacillus plantarum* ECGC 13110402 | 240 mg providing 2.6 × 10⁹ cells |
| Monacolin K | 2.0 mg |

### Formulation J

| Ingredient | Amount |
|---|---|
| *Lactobacillus plantarum* ECGC 13110402 | 240 mg providing 2.6 × 10⁹ cells |
| Alpha-linolenic acid (ALA) | 3.4 g |

### Formulation K

| Ingredient | Amount |
|---|---|
| *Lactobacillus plantarum* ECGC 13110402 | 240 mg providing 2.6 × 10⁹ cells |
| Alpha-linolenic acid (ALA) | 1 g |

### Formulation L

| Ingredient | Amount |
|---|---|
| *Lactobacillus plantarum* ECGC 13110402 | 240 mg providing 2.6 × 10⁹ cells |
| Alpha-linolenic acid (ALA) | 1 g |
| Omega-3 PUFAs | 4 g |
| Omega-6 PUFAs | 60 g |

### Formulation M

| Ingredient | Amount |
|---|---|
| *Lactobacillus plantarum* ECGC 13110402 | 240 mg providing 2.6 × 10⁹ cells |
| β-glucans | 3 g |

### Formulation N

| Ingredient | Amount |
|---|---|
| *Lactobacillus plantarum* ECGC 13110402 | 240 mg providing 2.6 × 10⁹ cells |
| Pectin | 9 g |

### Formulation O

| Ingredient | Amount |
|---|---|
| *Lactobacillus plantarum* ECGC 13110402 | 240 mg providing 2.6 × 10⁹ cells |
| Pectin | 1 g |

### Formulation P

| Ingredient | Amount |
|---|---|
| *Lactobacillus plantarum* ECGC 13110402 | 240 mg providing 2.6 × 10⁹ cells |
| Pectin | 0.75 g |

### Formulation Q

| Ingredient | Amount |
|---|---|
| *Lactobacillus plantarum* ECGC 13110402 | 240 mg providing 2.6 × 10⁹ cells |
| Chitosan | 3 g |

### Formulation R

| Ingredient | Amount |
|---|---|
| *Lactobacillus plantarum* ECGC 13110402 | 240 mg providing 2.6 × 10⁹ cells |
| Inulin | 20 g |

### Formulation S

| Ingredient | Amount |
|---|---|
| *Lactobacillus plantarum* ECGC 13110402 | 240 mg providing 2.6 × 10⁹ cells |
| Fructooligosaccharides (FOS) | 15 g |

### Formulation T

| Ingredient | Amount |
|---|---|
| *Lactobacillus plantarum* ECGC 13110402 | 240 mg providing 2.6 × 10⁹ cells |
| Galactooligosaccharides (GOS) | 15 g |

### Formulation U

| Ingredient | Amount |
|---|---|
| *Lactobacillus plantarum* ECGC 13110402 | 240 mg providing 2.6 × 10⁹ cells |
| simvastin | 7.5 mg |
| Monacolin K | 2.5 mg |
| β-glucans | 3 g |
| Fructooligosaccharides (FOS) | 15 g |

### Formulation V

| Ingredient | Amount |
|---|---|
| *Lactobacillus plantarum* ECGC 13110402 | 240 mg providing 2.6 × 10⁹ cells |
| simvastin | 7.5 mg |
| Alpha-linolenic acid (ALA) | 1 g |
| Omega-3 PUFAs | 4 g |
| Galactooligosaccharides (GOS) | 15 g |

### Formulation W

| Ingredient | Amount |
|---|---|
| *Lactobacillus plantarum* ECGC 13110402 | 240 mg providing 2.6 × 10⁹ cells |
| simvastin | 7.5 mg |
| Pytosterols | 2.5 g |
| Alpha-linolenic acid (ALA) | 1 g |
| Omega-3 PUFAs | 4 g |

### Formulation X

| Ingredient | Amount |
|---|---|
| *Lactobacillus plantarum* ECGC 13110402 | 240 mg providing 2.6 × 10⁹ cells |
| Pytosterols | 3 g |
| Pysllium | 7 g |
| Omega-3 PUFAs | 4 g |

### Formulation Y

| Ingredient | Amount |
|---|---|
| *Lactobacillus plantarum* ECGC 13110402 | 240 mg providing 2.6 × 10⁹ cells |
| β-glucans | 3 g |
| Glucomannan | 3 g |
| Omega-3 PUFAs | 4 g |

### Formulation Z

| Ingredient | Amount |
|---|---|
| *Lactobacillus plantarum* ECGC 13110402 | 240 mg providing 2.6 × 10⁹ cells |
| Pytosterols | 2.5 g |
| Pysllium | 7 g |
| Glucomannan | 3 g |
| Omega-3 PUFAs | 3 g |

It will be apparent to the skilled addressee that the above formulations can be formulated in a number of ways, such as in tables or as foodstuffs or food supplements and divided into multiple doses so as to achieve the stated daily doses.

### Biological Deposits

The application refers to the following indications of deposited biological material:

| | |
|---|---|
| Name: | European Collection of Cell Cultures |
| Address: | Public Health England Porton Down, |
| | National Collection of Type Cultures, |
| | PHE Culture Collections, Microbiological Services, |
| | Porton Down, |
| | Salisbury, |
| | SP4 0JG |
| | United Kingdom |
| Date: | 04 November 2013 |
| Accession Number: | 13110402 |
| Depositor: | Optibiotix Health Limited (which subsequently changed name to Optibiotix Limited on 04 August 2014) United Kin |

## Claims

1. A composition for use in the management, treatment or prevention of hypertension, the composition comprising *Lactobacillus plantarum* ECGC 13110402 and galactooligosaccharides (GOS), wherein the GOS is produced by the *Lactobacillus plantarum* strain.

2. The composition for use of claim 1, further comprising one or more lipid modifying active ingredients selected from the following: statins; stanols and sterols; monacolin K; fatty acids; niacin, potassium, soluble fibres selected from one or more of the following: β-glucans; pectin; glucomannan; psyllium and/or chitosan; and/or prebiotics selected from one or more of the following: inulin; fructooligosaccharides (FOS); chitooligosaccharides (COS); xylooligosaccharides (XOS); gentiooligosaccharides; cellobiose and/or soyabean oligosaccharides.

3. The composition for use according to claim 2, wherein the one or more active ingredients comprises:
a. a statin selected from one or more of the following: fluvastatin; pravastatin; simvastatin; atorvastatin; and/or rosuvastatin; and/or
b. a fatty acid selected from one or more of the following: alpha-linolenic acid; omega-3 polyunsaturated fatty acids; and/or omega-6 polyunsaturated fatty acids.

4. The composition for use according to any of claims 1 to 3, wherein the *Lactobacillus plantarum* ECGC 13110402 is present in a daily dose in the range of 2 × 10⁵ to 2 × 10¹² cells.

5. The composition for use according to any of claims 1 to 4, wherein the *Lactobacillus plantarum* ECGC 13110402 is present in a daily dose in the range of 200 mg to 300 mg of the active strain providing 2.6 × 10⁹ cells.

6. The composition for use according to any one of claims 3 to 5, wherein the one or more active ingredients comprises fluvastatin in a daily dose in the range of 20 to 80 mg; pravastin in the range of 10 to 40 mg; simvastin in a daily dose in the range of 10 to 80 mg; rosuvastin in a daily dose in the range of 5 to 40 mg; and/or atorvastin in a daily dose in the range of 10 to 80 mg.

7. The composition for use according to any one of claims 3 to 6, wherein the one or more active ingredients comprises plant stanols and sterols in a daily dose in the range of 0.7 g to 3 g.

8. The composition for use according to any one of claims 3 to 7, wherein the one or more active ingredients comprises monacolin K in a daily dose in the range of 2.5 to 10 mg.

9. The composition for use according to any one of claims 3 to 8, wherein the one or more active ingredients comprises fatty acids in a daily dose in the range of 0.1 to 3.5 g ALA; 1 to 4g Omega-3 PUFAs; and/or 1 to 60 g Omega-6 PUFAs.

10. The composition for use according to any one of claims 3 to 9, wherein the one or more active ingredients comprises a daily dose of β-glucans in the range of 2 to 10 g; a daily dose of pectin in the range of 2 to 9 g; a daily dose of pysllium in the range of 5 to 15 g and/or a daily dose of chitosan in the range of 1 to 3 g.

11. The composition for use according to any one of claims 3 to 10, wherein the one or more active ingredients comprises a daily dose of inulin in the range of 5 to 20 g; a daily dose of fructooligosaccharides (FOS) in the range of 5 to 15 g; and/or galactooligosaccharides (GOS) in the range of 10 to 15 g.

12. The composition for use according to any preceding claim, wherein the composition is encapsulated.

13. The composition for use according to any preceding claim, wherein the composition is in the form of a capsule, tablet, powder or a liquid.

## Patentansprüche

1. Zusammensetzung zur Verwendung beim Management, der Behandlung oder der Vorbeugung von Bluthochdruck, wobei die Zusammensetzung *Lactobacillus plantarum* ECGC 13110402 und Galactooligosaccharide (GOS) umfasst, wobei das GOS durch den *Lactobacillus* plantarum-Stamm erzeugt wird.

2. Zusammensetzung zur Verwendung nach Anspruch 1, die ferner einen oder mehrere lipidmodifizierende Wirkstoffe umfasst, ausgewählt aus den Folgenden: Statinen; Pflanzenbestandteilen und Sterolen; Monacolin K; Fettsäuren; Niacin, Kalium, lösliche Fasern, ausgewählt aus einem oder mehreren der Folgenden: β-Glucanen; Pektin; Glucomannan; Psyllium und/oder Chitosan; und/oder Präbiotika, ausgewählt aus einem oder mehreren der Folgenden: Inulin; Fructooligosacchariden (FOS); Chitooligosacchariden (COS); Xylooligosacchariden (XOS); Gentiooligosacchariden; Cellobiose und/oder Sojabohnen-Oligosacchariden.

3. Zusammensetzung zur Verwendung nach Anspruch 2, wobei der eine oder die mehreren Wirkstoffe umfassen:
a. ein Statin, ausgewählt aus einem oder mehreren der folgenden Stoffe: Fluvastatin, Pravastatin, Simvastatin, Atorvastatin und/oder Rosuvastatin, und/oder
b. eine Fettsäure, ausgewählt aus einer oder mehreren der Folgenden: Alpha-Linolensäure, mehrfach ungesättigten Omega-3-Fettsäuren und/oder mehrfach ungesättigten Omega-6-Fettsäuren.

4. Zusammensetzung zur Verwendung nach einem der Ansprüche 1 bis 3, wobei der *Lactobacillus plantarum* ECGC 13110402 in einer Tagesdosis im Bereich von 2 × 10⁵ bis 2 × 10¹² Zellen vorliegt.

5. Zusammensetzung zur Verwendung nach einem der Ansprüche 1 bis 4, wobei der *Lactobacillus plantarum* ECGC 13110402 in einer Tagesdosis im Bereich von 200 mg bis 300 mg des aktiven Stammes mit 2,6 × 10⁹ Zellen vorliegt.

6. Zusammensetzung zur Verwendung nach einem der Ansprüche 3 bis 5, wobei der eine oder die mehreren Wirkstoffe Fluvastatin in einer Tagesdosis im Bereich von 20 bis 80 mg, Pravastin im Bereich von 10 bis 40 mg, Simvastin in einer Tagesdosis im Bereich von 10 bis 80 mg, Rosuvastin in einer Tagesdosis im Bereich von 5 bis 40 mg und/oder Atorvastin in einer Tagesdosis im Bereich von 10 bis 80 mg umfassen.

7. Zusammensetzung zur Verwendung nach einem der Ansprüche 3 bis 6, wobei der eine oder die mehreren Wirkstoffe Pflanzenbestandteile und Sterole in einer Tagesdosis im Bereich von 0,7 g bis 3 g umfassen.

8. Zusammensetzung zur Verwendung nach einem der Ansprüche 3 bis 7, wobei der eine oder die mehreren Wirkstoffe Monacolin K in einer Tagesdosis im Bereich von 2,5 bis 10 mg umfassen.

9. Zusammensetzung zur Verwendung nach einem der Ansprüche 3 bis 8, wobei der eine oder die mehreren Wirkstoffe Fettsäuren in einer Tagesdosis im Bereich von 0,1 bis 3,5 g Alpha-Linolensäure, 1 bis 4 g mehrfach ungesättigte Omega-3-Fettsäuren und/oder 1 bis 60 g mehrfach ungesättigte Omega-6-Fettsäuren umfassen.

10. Zusammensetzung zur Verwendung nach einem der Ansprüche 3 bis 9, wobei der eine oder die mehreren Wirkstoffe eine Tagesdosis von β-Glucanen im Bereich von 2 bis 10 g, eine Tagesdosis von Pektin im Bereich von 2 bis 9 g, eine Tagesdosis von Pysllium im Bereich von 5 bis 15 g und/oder eine Tagesdosis von Chitosan im Bereich von 1 bis 3 g umfassen.

11. Zusammensetzung zur Verwendung nach einem der Ansprüche 3 bis 10, wobei der eine oder die mehreren Wirkstoffe eine Tagesdosis von Inulin im Bereich von 5 bis 20 g, eine Tagesdosis von Fructooligosacchariden (FOS) im Bereich von 5 bis 15 g und/oder Galactooligosacchariden (GOS) im Bereich von 10 bis 15 g umfassen.

12. Zusammensetzung zur Verwendung nach einem der vorstehenden Ansprüche, wobei die Zusammensetzung in Kapseln eingefüllt ist.

13. Zusammensetzung zur Verwendung nach einem der vorstehenden Ansprüche, wobei die Zusammensetzung in Form einer Kapsel, einer Tablette, eines Pulvers oder einer Flüssigkeit vorliegt.

## Revendications

1. Composition pour une utilisation dans la gestion, le traitement ou la prévention de l'hypertension, la composition comprenant *Lactobacillus plantarum* ECGC 13110402 et des galactooligosaccharides (GOS), dans laquelle le GOS est produit par la souche de *Lactobacillus plantarum.*

2. Composition pour une utilisation selon la revendication 1, comprenant en outre un ou plusieurs ingrédients actifs de modification de lipides sélectionnés parmi les éléments suivants : statines ; peuplements et stérols ; monacoline K ; acides gras ; niacine, potassium, fibres solubles sélectionnées parmi un ou plusieurs des éléments suivants : β-glucanes ; pectine ; glucomannane ; psyllium et/ou chitosane ; et/ou prébiotiques sélectionnés parmi un ou plusieurs des éléments suivants : inuline ; fructooligosaccharides (FOS) ; chitooligosaccharides (COS) ; xylooligosaccharides (XOS) ; gentiooligosaccharides ; cellobiose et/ou des oligosaccharides de soja.

3. Composition pour une utilisation selon la revendication 2, dans laquelle le ou les ingrédients actifs comprennent :
a. une statine sélectionnée parmi un ou plusieurs des éléments suivants : fluvastatine ; pravastatine ; simvastatine ; atorvastatine ; et/ou rosuvastatine ; et/ou
b. un acide gras sélectionné parmi un ou plusieurs des éléments suivants : acide alpha-linolénique ; acides gras polyinsaturés oméga-3 ; et/ou acides gras polyinsaturés oméga-6.

4. Composition pour une utilisation selon l'une quelconque des revendications 1 à 3, dans laquelle le *Lactobacillus plantarum* ECGC 13110402 est présent dans une dose quotidienne dans la plage de 2 × 10⁵ à 2 × 10¹² cellules.

5. Composition pour une utilisation selon l'une quelconque des revendications 1 à 4, dans laquelle le *Lactobacillus plantarum* ECGC 13110402 est présent dans une dose quotidienne dans la plage de 200 mg à 300 mg de la souche active fournissant 2,6 × 10⁹ cellules.

6. Composition pour une utilisation selon l'une quelconque des revendications 3 à 5, dans laquelle le ou les ingrédients actifs comprennent de la fluvastatine en une dose quotidienne dans la plage de 20 à 80 mg ; pravastine dans la plage de 10 à 40 mg ; simvastine en une dose quotidienne dans la plage de 10 à 80 mg ; rosuvastine en une dose quotidienne dans la plage de 5 à 40 mg ; et/ou atorvastine en une dose quotidienne dans la plage de 10 à 80 mg.

7. Composition pour une utilisation selon l'une quelconque des revendications 3 à 6, dans laquelle le ou les ingrédients actifs comprennent des peuplements végétaux et des stérols en une dose quotidienne dans la plage de 0,7 g à 3 g.

8. Composition pour une utilisation selon l'une quelconque des revendications 3 à 7, dans laquelle le ou les ingrédients actifs comprennent de la monacoline K en une dose quotidienne dans la plage de 2,5 à 10 mg.

9. Composition pour une utilisation selon l'une quelconque des revendications 3 à 8, dans laquelle le ou les ingrédients actifs comprennent des acides gras en une dose quotidienne dans la plage de 0,1 à 3,5 g d'ALA ; 1 à 4 g de PUFA Oméga-3 ; et/ou 1 à 60 g de PUFA Oméga-6.

10. Composition pour une utilisation selon l'une quelconque des revendications 3 à 9, dans laquelle le ou les ingrédients actifs comprennent une dose quotidienne de β-glucanes dans la plage de 2 à 10 g ; une dose quotidienne de pectine dans la plage de 2 à 9 g ; une dose quotidienne de pysllium dans la plage de 5 à 15 g et/ou une dose quotidienne de chitosane dans la plage de 1 à 3 g.

11. Composition pour une utilisation selon l'une quelconque des revendications 3 à 10, dans laquelle le ou les ingrédients actifs comprennent une dose quotidienne d'inuline dans la plage de 5 à 20 g ; une dose quotidienne de fructooligosaccharides (FOS) dans la plage de 5 à 15 g ; et/ou galactooligosaccharides (GOS) dans la plage de 10 à 15 g.

12. Composition pour une utilisation selon l'une quelconque des revendications précédentes, dans laquelle la composition est encapsulée.

13. Composition pour une utilisation selon l'une quelconque des revendications précédentes, dans laquelle la composition se présente sous la forme d'une capsule, d'un comprimé, d'une poudre ou d'un liquide.
